(19) Europäisches Patentamt
European Patent Office
Office européen des brevets

(11) **EP 4 270 307 A1**

(12) **EUROPEAN PATENT APPLICATION**
published in accordance with Art. 153(4) EPC

(43) Date of publication:
01.11.2023 Bulletin 2023/44

(21) Application number: 21911098.8

(22) Date of filing: 24.12.2021

(51) International Patent Classification (IPC):
*G06T 7/00* (2017.01)   *G16H 30/00* (2018.01)
*G16H 50/20* (2018.01)   *G06N 20/00* (2019.01)
*A61B 5/00* (2006.01)

(52) Cooperative Patent Classification (CPC):
A61B 5/00; G06N 20/00; G06T 7/00; G16H 30/00;
G16H 50/20

(86) International application number:
PCT/JP2021/048387

(87) International publication number:
WO 2022/138961 (30.06.2022 Gazette 2022/26)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 25.12.2020 JP 2020217839

(71) Applicant: FUJIFILM Corporation
Tokyo 106-8620 (JP)

(72) Inventor: LI, Yuanzhong
Ashigarakami-gun, Kanagawa 258-8577 (JP)

(74) Representative: Hughes, Andrea Michelle
Dehns Germany
Theresienstraße 6-8
80333 München (DE)

(54) **INFORMATION PROCESSING DEVICE, INFORMATION PROCESSING DEVICE OPERATING METHOD, AND INFORMATION PROCESSING DEVICE OPERATING PROGRAM**

(57) There is provided an information processing apparatus including: a processor; and a memory connected to or built in the processor, in which the processor is configured to generate a scatter diagram for a machine learning model that receives a plurality of types of input data and outputs output data according to the input data, by plotting, in a two-dimensional space in which two parameters which are set based on the plurality of types of input data are set as a horizontal axis and a vertical axis, marks representing a plurality of samples obtained by inputting the input data to the machine learning model, and display the scatter diagram, the input data, and a type of the output data on a display.

FIG. 23

**Description**

BACKGROUND OF THE INVENTION

1. Field of the Invention

A technique of the present disclosure relates to an information processing apparatus, an operation method of an information processing apparatus, and an operation program of an information processing apparatus.

2. Description of the Related Art

In a field of machine learning, so-called multimodal learning, in which a plurality of types of data are used as input data of a machine learning model, has recently attracted attention. For example, JP2019-530116A describes a technique for multimodal medical image processing of inputting genetic data and the like of a patient to a machine learning model in addition to a medical image such as a magnetic resonance imaging (MRI) image.

SUMMARY OF THE INVENTION

In the field of machine learning, there is a demand to verify a validity of output data which is output from the machine learning model according to the input data and to adopt the output data after satisfaction is obtained. As a method of verifying the validity of the output data, a method of referring to another sample similar to a target sample for verifying the validity of the output data is considered. However, in a case of multimodal learning, there are a plurality of types of input data, and as a result, it is difficult to recognize a similarity between samples. Thus, it is difficult to verify the validity of the output data.

One embodiment according to the technique of the present disclosure provides an information processing apparatus, an operation method of an information processing apparatus, and an operation program of an information processing apparatus capable of easily verifying the validity of output data which is output from a machine learning model in multimodal learning.

According to the present disclosure, there is provided an information processing apparatus including: a processor; and a memory connected to or built in the processor, in which the processor is configured to generate a scatter diagram for a machine learning model that receives a plurality of types of input data and outputs output data according to the input data, by plotting, in a two-dimensional space in which two parameters which are set based on the plurality of types of input data are set as a horizontal axis and a vertical axis, marks representing a plurality of samples obtained by inputting the input data to the machine learning model, and display the scatter diagram, the input data, and a type of the output data on a display.

Preferably, the processor is configured to display the scatter diagram in a form in which the marks are allowed to be selected, and display, in a case where the mark is selected, at least the input data of the sample corresponding to the selected mark.

Preferably, the processor is configured to display pieces of the input data and types of pieces of the output data of at least two samples in a comparable manner.

Preferably, the mark represents the type of the output data.

Preferably, the mark represents matching/mismatching between the output data and an actual result.

Preferably, the processor is configured to set, as the horizontal axis and the vertical axis, the parameters related to two pieces of the input data which are preset among the plurality of types of input data.

Preferably, the machine learning model is constructed by a method of deriving a contribution of each of the plurality of types of input data to the output data, and the processor is configured to set, as the horizontal axis and the vertical axis, the parameters related to pieces of the input data which have a first contribution and a second contribution among the plurality of types of input data.

Preferably, the machine learning model is constructed by a method according to any one of linear discriminant analysis or boosting.

Preferably, the processor is configured to set, as the horizontal axis and the vertical axis, the parameters related to two pieces of the input data which are designated by a user among the plurality of types of input data.

Preferably, the processor is configured to generate the scatter diagram using a t-distributed stochastic neighbor embedding method.

Preferably, the plurality of types of input data include feature amount data obtained by inputting target region images of a plurality of target regions extracted from an image to feature amount derivation models prepared corresponding to the plurality of target regions, respectively.

Preferably, the feature amount derivation model includes at least one of an auto-encoder, a single-task convolutional neural network for class discrimination, or a multi-task convolutional neural network for class discrimination.

Preferably, the image is a medical image, the target regions are anatomical regions of an organ, and the machine learning model outputs, as the output data, an opinion of a disease.

Preferably, the plurality of types of input data include disease-related information related to the disease.

Preferably, the organ is a brain, and the disease is dementia. In this case, preferably, the anatomical regions include at least one of a hippocampus or a frontotemporal lobe.

According to the present disclosure, there is provided an operation method of an information processing apparatus, the method including: generating a scatter diagram for a machine learning model that receives a plurality of types of input data and outputs output data according to the input data, by plotting, in a two-dimensional space in which two parameters which are set based on the plurality of types of input data are set as a horizontal axis and a vertical axis, marks representing a plurality of samples obtained by inputting the input data to the machine learning model; and displaying the scatter diagram, the input data, and a type of the output data on a display.

According to the present disclosure, there is provided an operation program of an information processing apparatus, the program causing a computer to execute a process including: generating a scatter diagram for a machine learning model that receives a plurality of types of input data and outputs output data according to the input data, by plotting, in a two-dimensional space in which two parameters which are set based on the plurality of types of input data are set as a horizontal axis and a vertical axis, marks representing a plurality of samples obtained by inputting the input data to the machine learning model; and displaying the scatter diagram, the input data, and a type of the output data on a display.

According to the technique of the present disclosure, it is possible to provide an information processing apparatus, an operation method of an information processing apparatus, and an operation program of an information processing apparatus capable of easily verifying the validity of output data which is output from a machine learning model in multimodal learning.

BRIEF DESCRIPTION OF THE DRAWINGS

Fig. 1 is a diagram illustrating a medical system including a diagnosis support device.
Fig. 2 is a diagram illustrating dementia-related information.
Fig. 3 is a block diagram illustrating a computer including the diagnosis support device.
Fig. 4 is a block diagram illustrating a processing unit of a CPU of the diagnosis support device.
Fig. 5 is a diagram illustrating processing of a normalization unit.
Fig. 6 is a diagram illustrating processing of an extraction unit.
Fig. 7 is a diagram illustrating processing of a feature amount derivation unit.
Fig. 8 is a diagram illustrating processing of a dementia opinion derivation unit.
Fig. 9 is a diagram illustrating a configuration of an auto-encoder, a configuration of a single-task convolutional neural network for class discrimination, and a structure of a feature amount derivation model.
Fig. 10 is a diagram explaining convolution processing.
Fig. 11 is a diagram illustrating a configuration of operation data.
Fig. 12 is a diagram explaining pooling processing.
Fig. 13 is a diagram illustrating a detailed configuration of an output unit.
Fig. 14 is a diagram illustrating an outline of processing in a learning phase of the auto-encoder and the single-task convolutional neural network for class discrimination.
Fig. 15 is a graph illustrating a change of a weight given to a loss of the auto encoder.
Fig. 16 is a diagram illustrating an outline of processing in a learning phase of a dementia opinion derivation model.
Fig. 17 is a diagram illustrating sample information.
Fig. 18 is a diagram illustrating contribution information and axis setting information.
Fig. 19 is a diagram illustrating a state where a scatter diagram is generated.
Fig. 20 is a diagram illustrating a first display screen.
Fig. 21 is a diagram illustrating a second display screen.
Fig. 22 is a diagram illustrating a verification screen.
Fig. 23 is a diagram illustrating a verification screen.
Fig. 24 is a flowchart illustrating a processing procedure of the diagnosis support device.
Fig. 25 is a diagram illustrating another example of the dementia opinion derivation model.
Fig. 26 is a diagram illustrating a form in which parameters related to two pieces of input data designated by a user are set as a horizontal axis and a vertical axis.
Fig. 27 is a diagram illustrating a form of generating a scatter diagram by using a t-distributed stochastic neighbor embedding method.

Fig. 28 is a diagram illustrating a configuration of an auto-encoder and a structure of a feature amount derivation model.

Fig. 29 is a diagram illustrating an outline of processing in a learning phase of the auto-encoder.

Fig. 30 is a diagram illustrating processing of a dementia opinion derivation unit according to a second embodiment.

Fig. 31 is a diagram illustrating a configuration of a single-task convolutional neural network for class discrimination and a structure of a feature amount derivation model.

Fig. 32 is a diagram illustrating an outline of processing in a learning phase of the single-task convolutional neural network for class discrimination.

Fig. 33 is a diagram illustrating a configuration of a multi-task convolutional neural network for class discrimination and a structure of a feature amount derivation model.

Fig. 34 is a diagram illustrating an outline of processing in a learning phase of the multi-task convolutional neural network for class discrimination.

Fig. 35 is a diagram illustrating processing of a feature amount derivation unit according to a fifth embodiment.

Fig. 36 is a diagram illustrating another example of dementia opinion information.

Fig. 37 is a diagram illustrating another example of dementia opinion information.

Fig. 38 is a diagram illustrating still another example of dementia opinion information.

DESCRIPTION OF THE PREFERRED EMBODIMENTS

[First Embodiment]

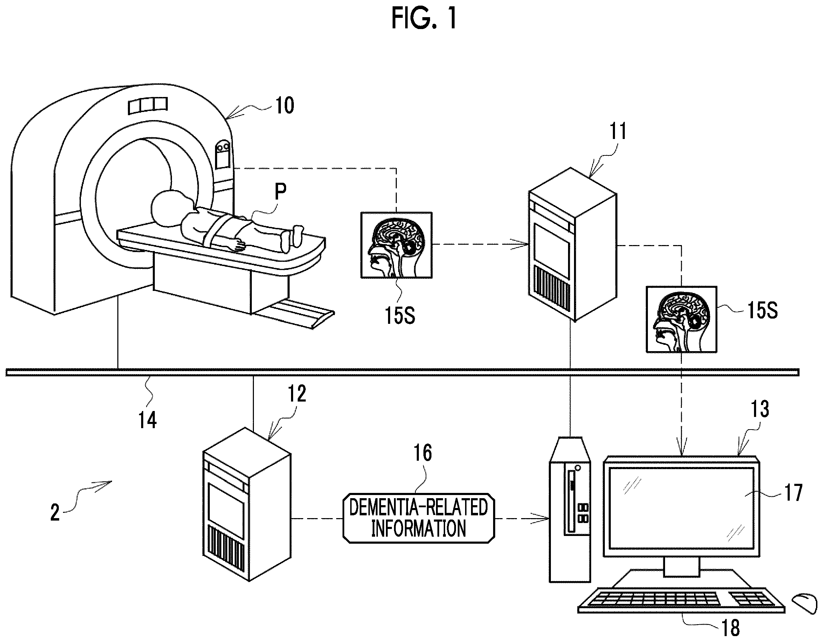

As illustrated in Fig. 1 as an example, a medical system 2 includes an MRI apparatus 10, a picture archiving and communication system (PACS) server 11, an electronic medical record server 12, and a diagnosis support device 13. The MRI apparatus 10, the PACS server 11, the electronic medical record server 12, and the diagnosis support device 13 are connected to a local area network (LAN) 14 provided in a medical facility, and can communicate with each other via the LAN 14.

The MRI apparatus 10 images a head of a patient P and outputs a head MRI image 15. The head MRI image 15 is voxel data representing a three-dimensional shape of the head of the patient P. In Fig. 1, a head MRI image 15S having a sagittal cross section is illustrated. The MRI apparatus 10 transmits the head MRI image 15 to the PACS server 11. The PACS server 11 stores and manages the head MRI image 15 from the MRI apparatus 10. The electronic medical record server 12 stores and manages an electronic medical record of the patient P. The electronic medical record includes dementia-related information 16 related to dementia of the patient P. The head MRI image 15 is an example of an "image" and a "medical image" according to the technique of the present disclosure. In addition, dementia is an example of "disease" according to the technique of the present disclosure, and the dementia-related information 16 is an example of "disease-related information" according to the technique of the present disclosure.

The diagnosis support device 13 is, for example, a desktop personal computer, and includes a display 17 and an input device 18. The input device 18 is a keyboard, a mouse, a touch panel, a microphone, or the like. A doctor transmits a distribution request of the head MRI image 15 of the patient P to the PACS server 11 by operating the input device 18. The PACS server 11 searches for the head MRI image 15 of the patient P that is requested to be distributed, and distributes the head MRI image 15 to the diagnosis support device 13. In addition, the doctor transmits a distribution request of the dementia-related information 16 of the patient P to the electronic medical record server 12. The electronic medical record server 12 searches for the dementia-related information 16 of the patient P that is requested to be distributed, and distributes the dementia-related information 16 of the patient P to the diagnosis support device 13. The diagnosis support device 13 displays the head MRI image 15 distributed from the PACS server 11 and the dementia-related information 16 distributed from the electronic medical record server 12 on the display 17. The doctor observes a brain of the patient P appearing in the head MRI image 15, and performs dementia diagnosis on the patient P while referring to the dementia-related information 16. The diagnosis support device 13 is an example of an "information processing apparatus" according to the technique of the present disclosure. In addition, the brain is an example of an "organ" according to the technique of the present disclosure. Further, the doctor is an example of a "user" according to the technique of the present disclosure. In Fig. 1, only one MRI apparatus 10 and one diagnosis support device 13 are illustrated. On the other hand, a plurality of MRI apparatuses 10 and a plurality of diagnosis support devices 13 may be provided.

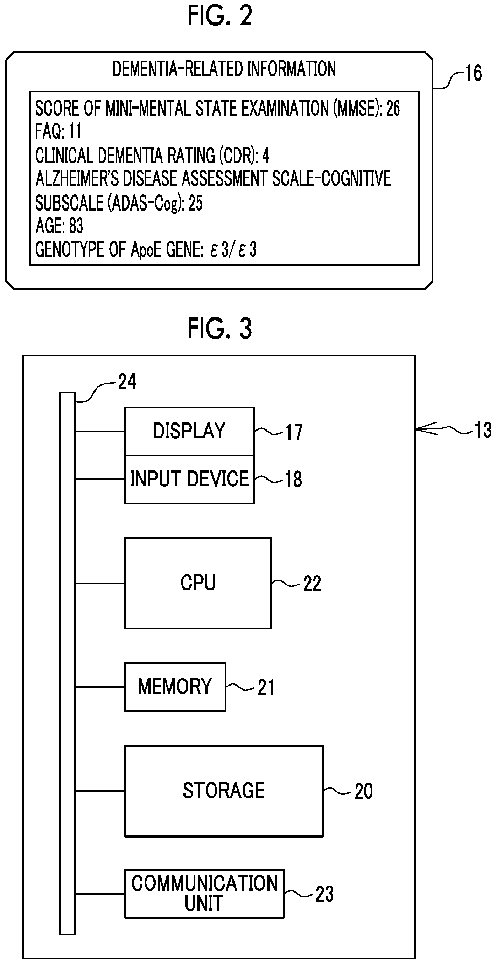

As illustrated in Fig. 2 as an example, the dementia-related information 16 includes a score of a mini-mental state examination (hereinafter, abbreviated as MMSE), a functional activities questionnaire (FAQ), a clinical dementia rating (hereinafter, abbreviated as CDR), and a score of a dementia test such as Alzheimer's disease assessment scale-cognitive subscale (hereinafter, abbreviated as ADAS-Cog).

In addition, the dementia-related information 16 includes an age of the patient P and a genotype of an ApoE gene. The genotype of the ApoE gene is a combination of two types among three types of ApoE genes of ε2, ε3, and ε4 (ε2 and ε3, ε3 and ε4, and the like). A risk of development of the Alzheimer's disease having a genotype including one or two ε4 (ε2 and ε4, ε4 and ε4, and the like) is approximately 3 times to 12 times a risk of development of the Alzheimer's disease having a genotype without ε4 (ε2 and ε3, ε3 and ε3, and the like).

In addition to these scores, a score of a dementia test such as a score of Hasegawa dementia scale, a score of a rivermead Behavioural memory test (RBMT), and activities of daily living (ADL) may be included in the dementia-related information 16. In addition, test results of a spinal fluid test, such as an amyloid β measurement value, a tau protein measurement value, and the like, may be included in the dementia-related information 16. Further, test results of a blood test, such as an apolipoprotein measurement value, a complement protein measurement value, and a transthyretin measurement value, may be included in the dementia-related information 16. In addition, the dementia-related information 16 may include a gender and a medical history of the patient P, whether or not the patient P has a relative who develops dementia, and the like.

As illustrated in Fig. 3 as an example, a computer including the diagnosis support device 13 includes a storage 20, a memory 21, a central processing unit (CPU) 22, and a communication unit 23, in addition to the display 17 and the input device 18. The components are connected to each other via a bus line 24. The CPU 22 is an example of a "processor" according to the technique of the present disclosure.

The storage 20 is a hard disk drive that is built in the computer including the diagnosis support device 13 or is connected via a cable or a network. Alternatively, the storage 20 is a disk array in which a plurality of hard disk drives are connected in series. The storage 20 stores a control program such as an operating system, various types of application programs, and various types of data associated with the programs. A solid state drive may be used instead of the hard disk drive.

The memory 21 is a work memory which is necessary to execute processing by the CPU 22. The CPU 22 loads the program stored in the storage 20 into the memory 21, and executes processing according to the program. Thereby, the CPU 22 collectively controls each unit of the computer. The communication unit 23 controls transmission of various types of information to an external apparatus such as the PACS server 11. The memory 21 may be built in the CPU 22.

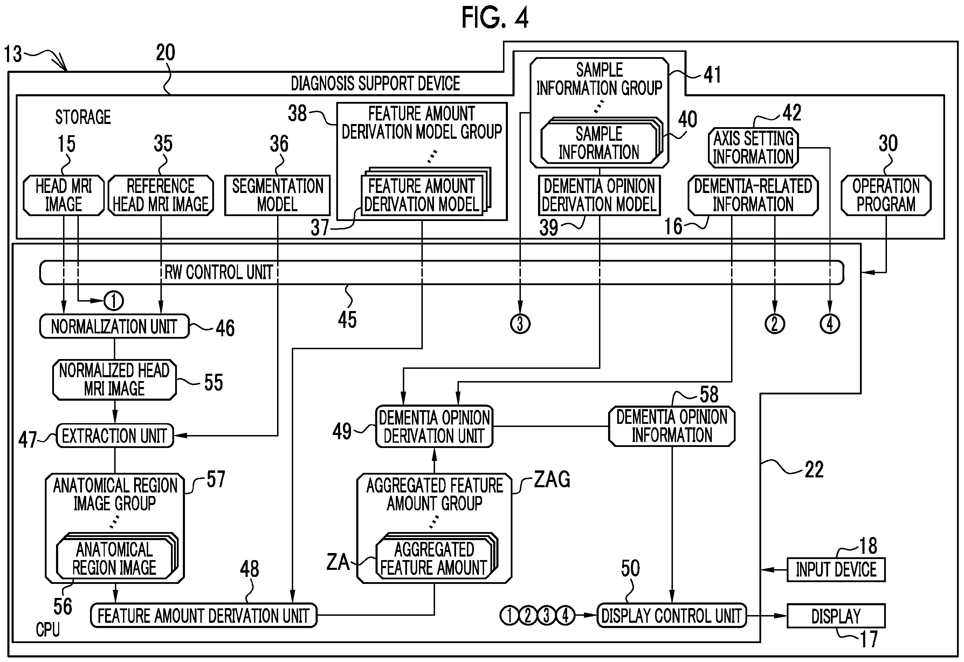

As illustrated in Fig. 4 as an example, an operation program 30 is stored in the storage 20 of the diagnosis support device 13. The operation program 30 is an application program for causing the computer to function as the information processing apparatus according to the technique of the present disclosure. That is, the operation program 30 is an example of "an operation program of the information processing apparatus" according to the technique of the present disclosure. The storage 20 also stores the head MRI image 15, the dementia-related information 16, a reference head MRI image 35, and a segmentation model 36. Further, the storage 20 also stores a feature amount derivation model group 38 including a plurality of feature amount derivation models 37, a dementia opinion derivation model 39, a sample information group 41 including a plurality of pieces of sample information 40, and axis setting information 42.

In a case where the operation program 30 is started, the CPU 22 of the computer including the diagnosis support device 13 functions as a read/write (hereinafter, abbreviated as RW) control unit 45, a normalization unit 46, an extraction unit 47, a feature amount derivation unit 48, a dementia opinion derivation unit 49, and a display control unit 50, in cooperation with the memory 21 and the like.

The RW control unit 45 controls storing of various types of data in the storage 20 and reading of various types of data in the storage 20. For example, the RW control unit 45 receives the head MRI image 15 from the PACS server 11, and stores the received head MRI image 15 in the storage 20. In addition, the RW control unit 45 receives the dementia-related information 16 from the electronic medical record server 12, and stores the received dementia-related information 16 in the storage 20. In Fig. 4, only one head MRI image 15 and one piece of dementia-related information 16 are stored in the storage 20. On the other hand, a plurality of head MRI images 15 and a plurality of pieces of dementia-related information 16 may be stored in the storage 20.

The RW control unit 45 reads, from the storage 20, the head MRI image 15 and the dementia-related information 16 of the patient P designated by the doctor for diagnosing dementia. The RW control unit 45 outputs the head MRI image 15 which is read to the normalization unit 46 and the display control unit 50. In addition, the RW control unit 45 outputs the dementia-related information 16 which is read to the dementia opinion derivation unit 49 and the display control unit 50.

The RW control unit 45 reads the reference head MRI image 35 from the storage 20, and outputs the reference head MRI image 35 which is read to the normalization unit 46. The RW control unit 45 reads the segmentation model 36 from the storage 20, and outputs the segmentation model 36 which is read to the extraction unit 47. The RW control unit 45 reads the feature amount derivation model group 38 from the storage 20, and outputs the feature amount derivation model group 38 which is read to the feature amount derivation unit 48. The RW control unit 45 reads the dementia opinion derivation model 39 from the storage 20, and outputs the dementia opinion derivation model 39 which is read to the dementia opinion derivation unit 49. The RW control unit 45 reads the sample information group 41 from the storage 20, and outputs the sample information group 41 which is read to the display control unit 50. Further, the RW control unit 45 reads the axis setting information 42 from the storage 20, and outputs the axis setting information 42 which is read to the display control unit 50.

The normalization unit 46 performs normalization processing of matching the head MRI image 15 with the reference head MRI image 35, and sets the head MRI image 15 as a normalized head MRI image 55. The normalization unit 46 outputs the normalized head MRI image 55 to the extraction unit 47.

The reference head MRI image 35 is a head MRI image in which a brain having a reference shape, a reference size, and a reference shade (pixel value) appears. The reference head MRI image 35 is, for example, an image generated by averaging head MRI images 15 of a plurality of healthy persons, or an image generated by computer graphics.

The extraction unit 47 inputs the normalized head MRI image 55 to the segmentation model 36. The segmentation model 36 is a machine learning model that performs so-called semantic segmentation of assigning a label representing each of anatomical regions of a brain, such as a left hippocampus, a right hippocampus, a left frontotemporal lobe, and a right frontotemporal lobe, to each pixel of the brain appearing in the normalized head MRI image 55. The extraction unit 47 extracts images 56 of a plurality of anatomical regions of the brain (hereinafter, referred to as anatomical region images) from the normalized head MRI image 55 based on the labels assigned by the segmentation model 36. The extraction unit 47 outputs an anatomical region image group 57 including the plurality of anatomical region images 56 for each of the plurality of anatomical regions to the feature amount derivation unit 48. The anatomical region is an example of a "target region" according to the technique of the present disclosure. In addition, the anatomical region image 56 is an example of a "target region image" according to the technique of the present disclosure.

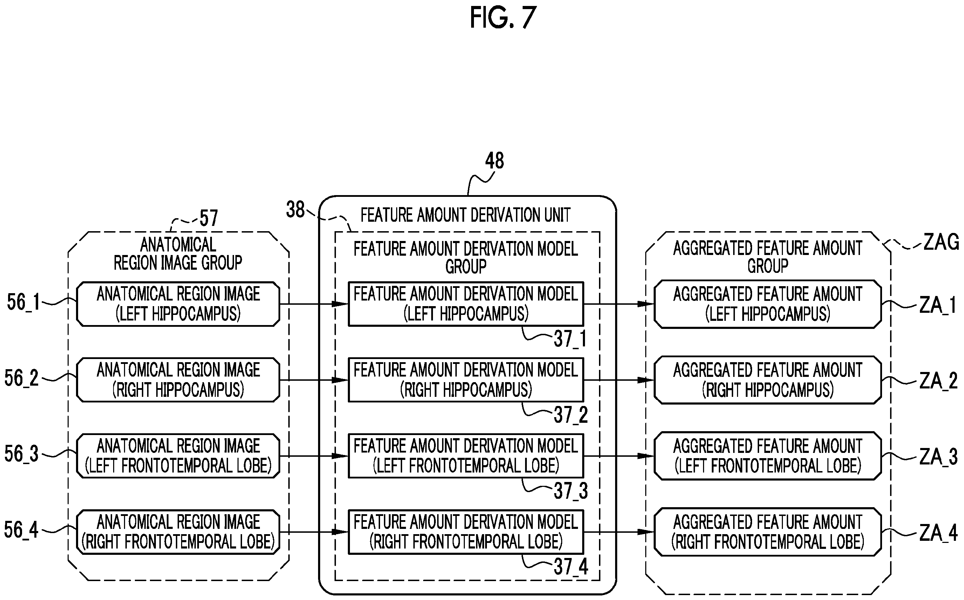

One feature amount derivation model 37 is prepared for each of the anatomical region images 56 (refer to Fig. 7). The feature amount derivation unit 48 inputs the anatomical region image 56 to the corresponding feature amount derivation model 37. In addition, an aggregated feature amount ZA is output from the feature amount derivation model 37. The feature amount derivation unit 48 outputs an aggregated feature amount group ZAG including a plurality of aggregated feature amounts ZA corresponding to the plurality of anatomical region images 56 to the dementia opinion derivation unit 49. The aggregated feature amount ZA is an example of "feature amount data" according to the technique of the present disclosure.

The dementia opinion derivation unit 49 inputs the dementia-related information 16 and the aggregated feature amount group ZAG to the dementia opinion derivation model 39. In addition, dementia opinion information 58 representing a dementia opinion is output from the dementia opinion derivation model 39. The dementia opinion derivation unit 49 outputs the dementia opinion information 58 to the display control unit 50. The dementia opinion derivation model 39 is an example of a "machine learning model" according to the technique of the present disclosure. In addition, the MMSE score, the CDR, the age, and the like included in the dementia-related information 16 and the plurality of aggregated feature amounts ZA included in the aggregated feature amount group ZAG are examples of "input data" according to the technique of the present disclosure. Further, the dementia opinion information 58 is an example of "output data" according to the technique of the present disclosure.

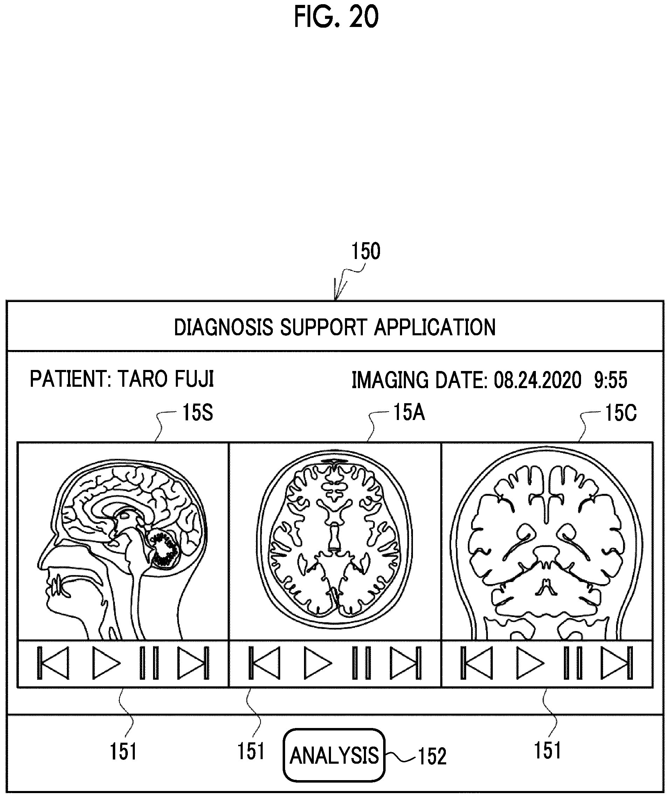

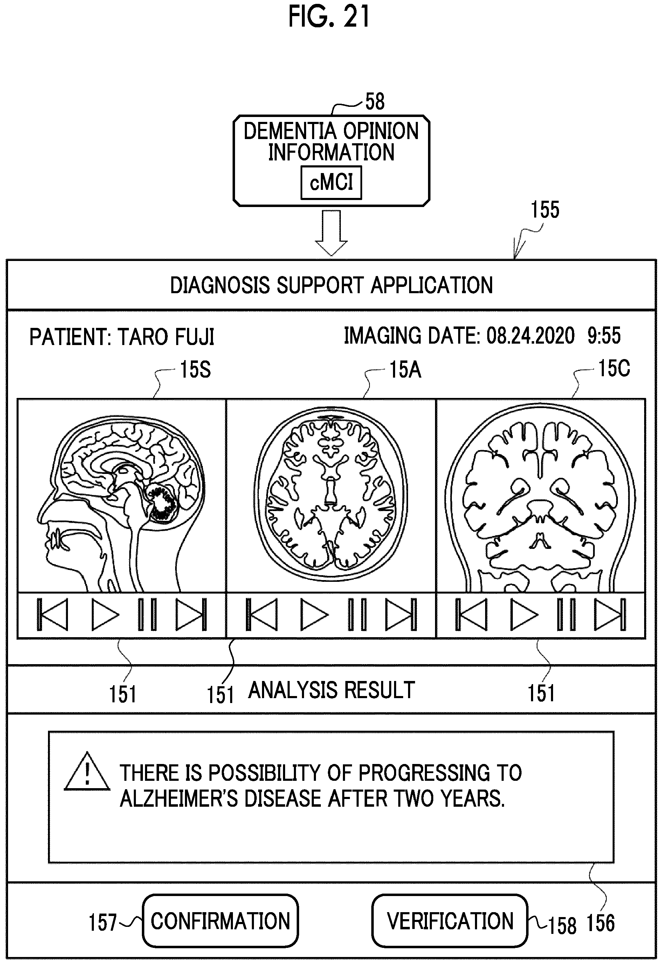

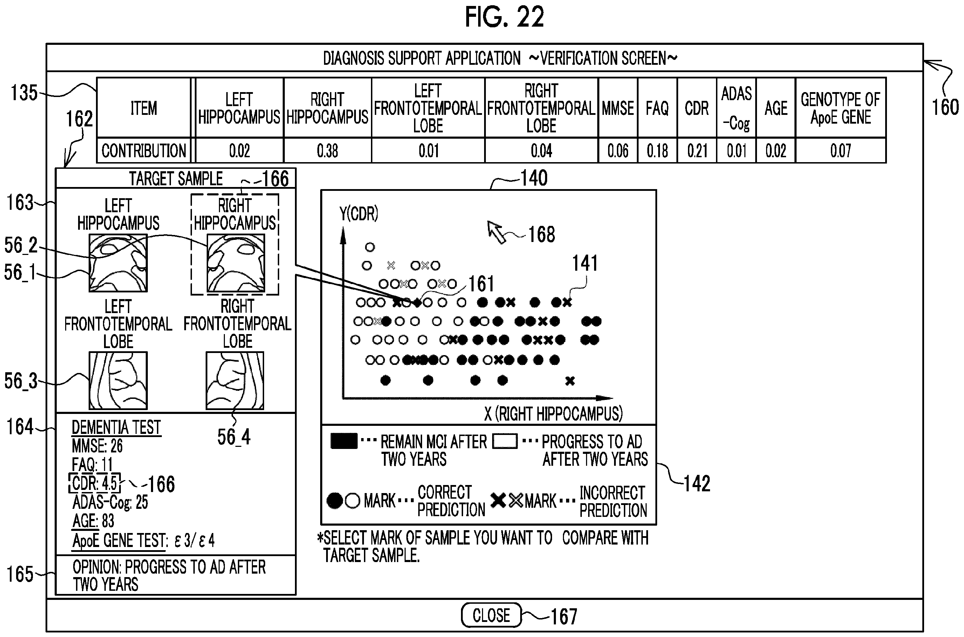

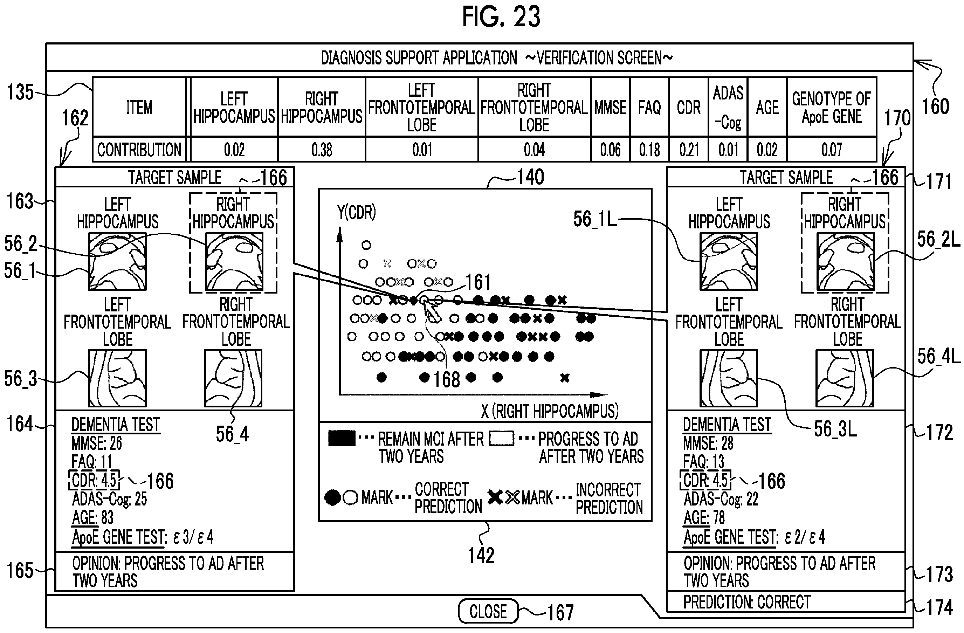

The display control unit 50 controls a display of various screens on the display 17. The various screens include a first display screen 150 (refer to Fig. 20) for instructing analysis by the segmentation model 36, the feature amount derivation model 37, and the dementia opinion derivation model 39, a second display screen 155 (refer to Fig. 21) for displaying the dementia opinion information 58, a verification screen 160 (refer to Fig. 22 and Fig. 23) for verifying a validity of the dementia opinion information 58, and the like.

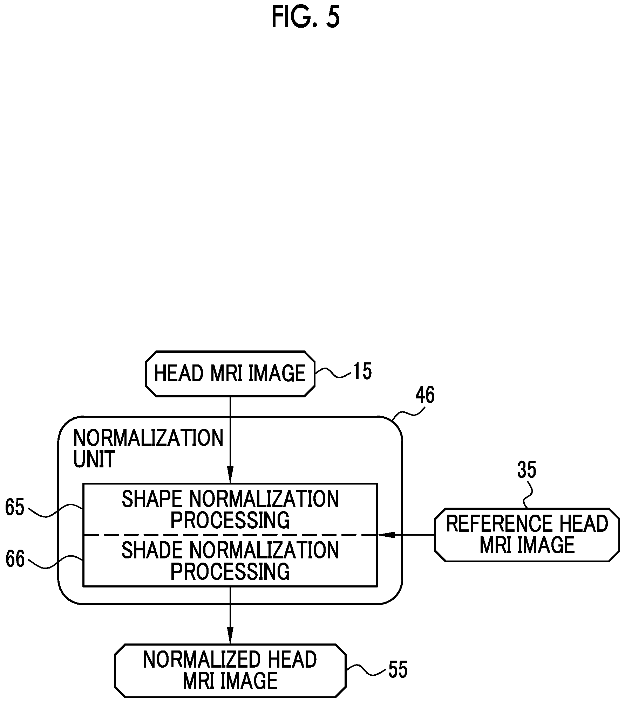

As illustrated in Fig. 5 as an example, the normalization unit 46 performs, as normalization processing, shape normalization processing 65 and shade normalization processing 66 on the head MRI image 15. The shape normalization processing 65 is processing of extracting, for example, landmarks serving as references for registration from the head MRI image 15 and the reference head MRI image 35, and performing parallel displacement, rotation, and/or enlargement/reduction of the head MRI image 15 in accordance with the reference head MRI image 35 such that a correlation between the landmark of the head MRI image 15 and the landmark of the reference head MRI image 35 is maximized. The shade normalization processing 66 is, for example, processing of correcting a shade histogram of the head MRI image 15 in accordance with a shade histogram of the reference head MRI image 35.

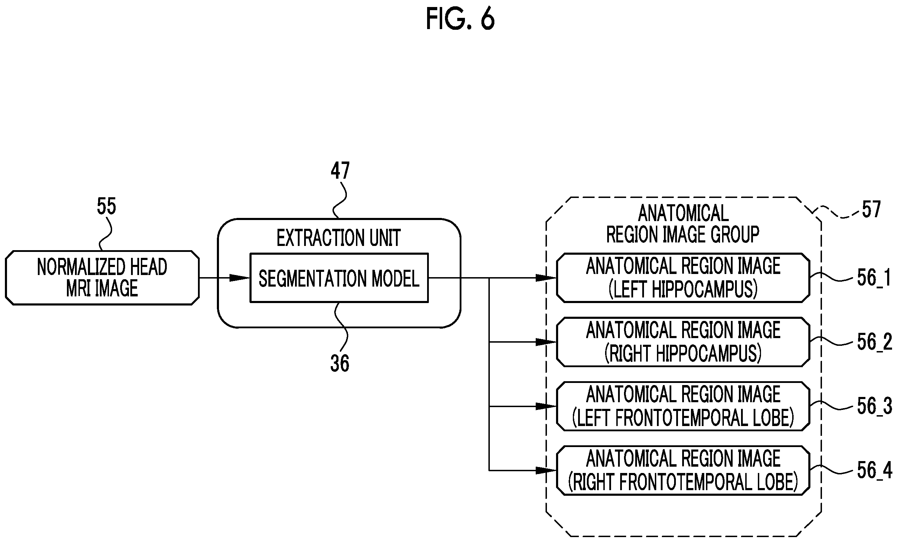

As illustrated in Fig. 6 as an example, the extraction unit 47 extracts, as the anatomical region images 56, the anatomical region image 56_1 of a left hippocampus, the anatomical region image 56_2 of a right hippocampus, the anatomical region image 56_3 of a left frontotemporal lobe, and the anatomical region image 56_4 of a right frontotemporal lobe. As described above, preferably, the anatomical region includes at least one of a hippocampus or a frontotemporal lobe. More preferably, the anatomical region includes all of a hippocampus and a frontotemporal lobe. The frontotemporal lobe means a front portion of a temporal lobe. In addition to the parts, the extraction unit 47 may extract anatomical region images 56 of anatomical regions such as a frontal lobe, an occipital lobe, a thalamus, a hypothalamus, an amygdala, a pituitary gland, a mamillary body, a corpus callosum, a fornix, and a lateral ventricle. For the extraction of the anatomical regions by the extraction unit 47 using the segmentation model 36, for example, a method described in the following literature is used.

<Patrick McClure, etc., Knowing What You Know in Brain Segmentation Using Bayesian Deep Neural Networks, Front. Neuroinform., 17 October 2019.>

As illustrated in Fig. 7 as an example, the feature amount derivation unit 48 inputs the anatomical region image 56_1 of the left hippocampus to the feature amount derivation model 37_1 of the left hippocampus, and outputs the aggregated feature amount ZA_1 of the left hippocampus from the feature amount derivation model 37_1 of the left hippocampus.

Similarly, the feature amount derivation unit 48 inputs the anatomical region image 56_2 of the right hippocampus to the feature amount derivation model 37_2 of the right hippocampus, and inputs the anatomical region image 56_3 of the left frontotemporal lobe to the feature amount derivation model 37_3 of the left frontotemporal lobe. In addition, the feature amount derivation unit 48 inputs the anatomical region image 56_4 of the right frontotemporal lobe to the feature amount derivation model 37_4 of the right frontotemporal lobe. Further, the feature amount derivation unit 48 outputs the aggregated feature amount ZA_2 of the right hippocampus from the feature amount derivation model 37_2 of the right hippocampus, and outputs the aggregated feature amount ZA_3 of the left frontotemporal lobe from the feature amount derivation model 37_3 of the left frontotemporal lobe. In addition, the feature amount derivation unit 48 outputs the aggregated feature amount ZA_4 of the right frontotemporal lobe from the feature amount derivation model 37_4 of the right frontotemporal lobe. As described above, the plurality of anatomical region images 56 are respectively input to the corresponding feature amount derivation models 37. Thereby, the plurality of aggregated feature amounts ZA for each of the anatomical region images 56 are output from the feature amount derivation models 37.

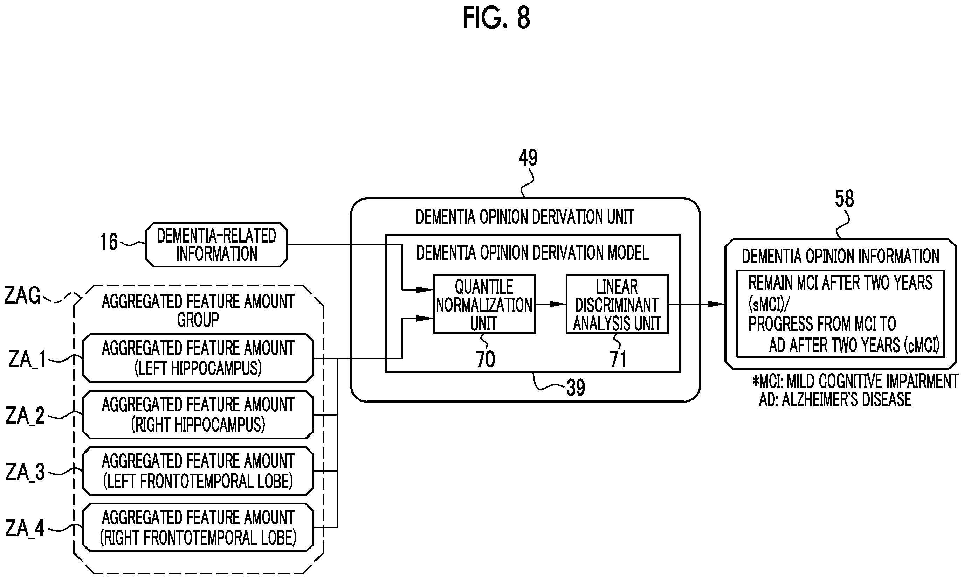

As illustrated in Fig. 8 as an example, the dementia opinion derivation unit 49 inputs the dementia-related information 16 and the aggregated feature amount group ZAG to the dementia opinion derivation model 39. In addition, the dementia opinion derivation unit 49 outputs, as the dementia opinion information 58, information indicating that the patient P who currently has mild cognitive impairment (MCI) remains mild cognitive impairment after two years or progresses to Alzheimer's disease (AD) after two years. In the following description, "a state where the patient P who currently has mild cognitive impairment remains mild cognitive impairment even after two years" will be referred to as stable MCI (sMCI). In addition, "a state where the patient P who currently has mild cognitive impairment progresses to Alzheimer's disease (AD) after two years" will be referred to as convert MCI (cMCI).

The dementia opinion derivation model 39 includes a quantile normalization unit 70 and a linear discriminant analysis unit 71. The dementia-related information 16 and the aggregated feature amount group ZAG are input to the quantile normalization unit 70. The quantile normalization unit 70 performs quantile normalization of converting the MMSE score included in the dementia-related information 16 and the plurality of aggregated feature amounts ZA included in the aggregated feature amount group ZAG into data according to a normal distribution, in order to handle the MMSE score and the plurality of aggregated feature amounts ZA in the same sequence. The linear discriminant analysis unit 71 performs linear discriminant analysis on the dementia-related information 16 and the aggregated feature amount group ZAG after the quantile normalization processing, and outputs dementia opinion information 58 as a result of the linear discriminant analysis. That is, the dementia opinion derivation model 39 is constructed by a linear discriminant analysis method.

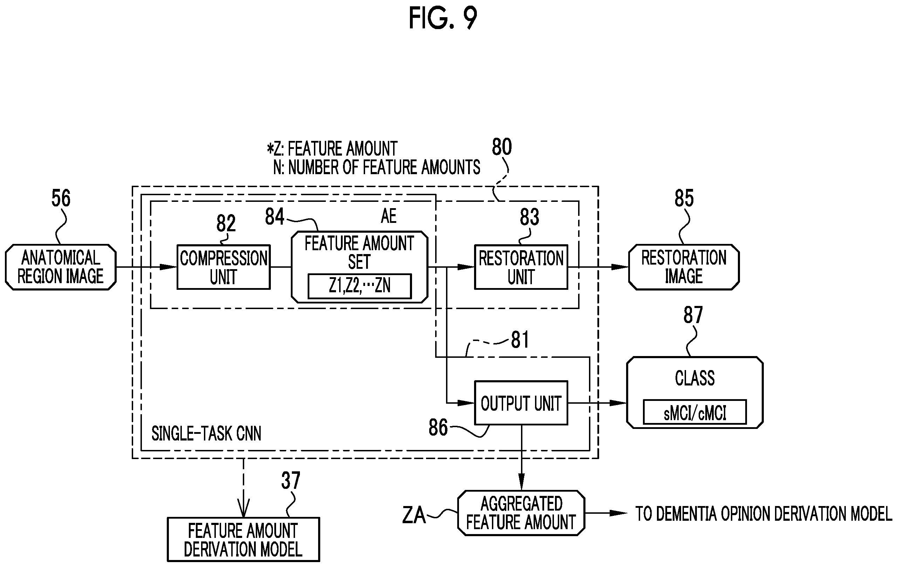

As illustrated in Fig. 9 as an example, as the feature amount derivation model 37, a model obtained by combining an auto encoder (hereinafter, abbreviated as AE) 80 and a single-task convolutional neural network for class discrimination (hereinafter, abbreviated as a single-task CNN) 81 is used. The AE 80 includes a compression unit 82 and a restoration unit 83. The anatomical region image 56 is input to the compression unit 82. The compression unit 82 converts the anatomical region image 56 into a feature amount set 84. The feature amount set 84 includes a plurality of feature amounts Z1, Z2, ..., ZN. N is the number of feature amounts, and is, for example, several tens to hundreds of thousands. The compression unit 82 transmits the feature amount set 84 to the restoration unit 83. The restoration unit 83 generates a restoration image 85 of the anatomical region image 56 from the feature amount set 84.

The single-task CNN 81 includes a compression unit 82 and an output unit 86. That is, the compression unit 82 is shared by the AE 80 and the single-task CNN 81. The compression unit 82 transmits the feature amount set 84 to the output unit 86. The output unit 86 outputs one class 87 based on the feature amount set 84. In Fig. 9, the output unit 86 outputs, as the class 87, a determination result of sMCI or cMCI. In addition, the output unit 86 outputs the aggregated feature amounts ZA obtained by aggregating the plurality of feature amounts Z included in the feature amount set 84.

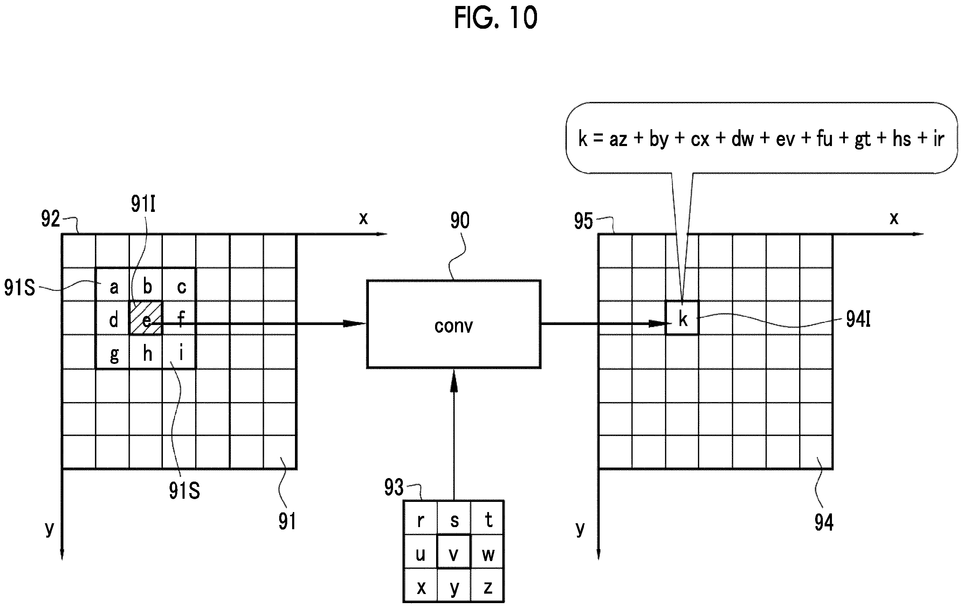

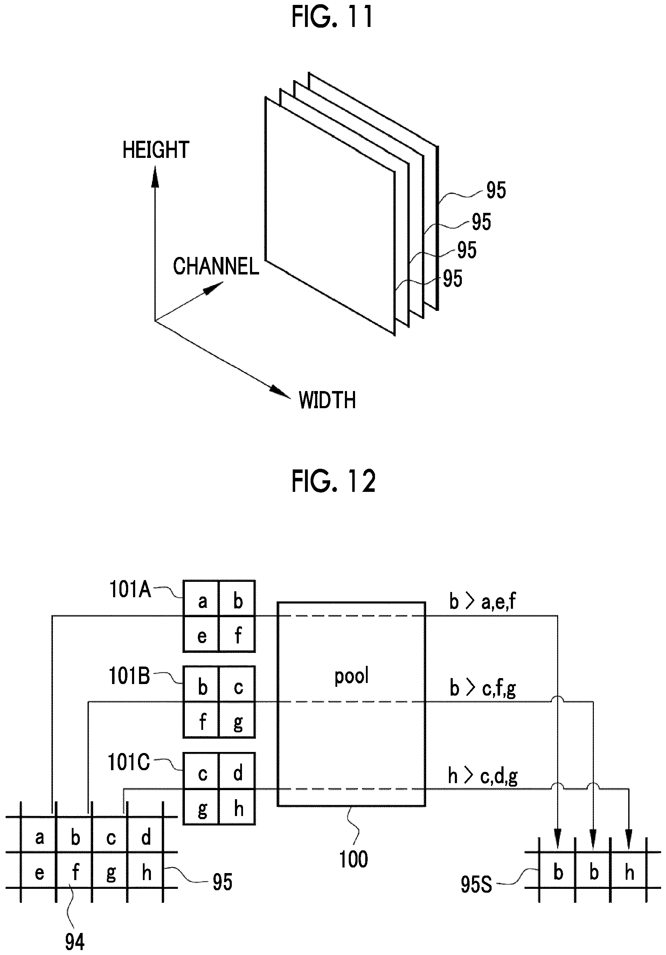

As an example, the compression unit 82 converts the anatomical region image 56 into the feature amount set 84 by performing a convolution operation as illustrated in Fig. 10. Specifically, the compression unit 82 includes a convolutional layer 90 represented by "convolution (abbreviated as conv)". The convolutional layer 90 applies, for example, a 3 × 3 filter 93 to the target data 92 including a plurality of elements 91 which are two-dimensionally arranged. In addition, the convolutional layer 90 performs convolution of an element value e of an element of interest 91I, which is one of the elements 91, and element values a, b, c, d, f, g, h, and i of eight elements 91S adjacent to the element of interest 91I. The convolutional layer 90 sequentially performs a convolution operation on each of the elements 91 of the target data 92 while shifting the element of interest 91I by one element, and outputs element values of elements 94 of operation data 95. Thereby, similarly to the target data 92, the operation data 95 including a plurality of elements 94 which are two-dimensionally arranged is obtained. The target data 92 that is first input to the convolutional layer 90 is the anatomical region image 56, and thereafter, reduction operation data 95S (refer to Fig. 12) to be described later is input to the convolutional layer 90 as the target data 92.

In a case where it is assumed that coefficients of the filter 93 are r, s, t, u, v, w, x, y, and z, an element value k of an element 941 of the operation data 95 corresponding to the element of interest 911 is obtained, for example, by calculating the following equation (1), the element value k being a result of the convolution operation on the element of interest 911.

$$k = az + by + cx + dw + ev + fu + gt + hs + ir \cdots (1)$$

One piece of the operation data 95 is output for one filter 93. In a case where a plurality of types of filters 93 are applied to one piece of the target data 92, the operation data 95 is output for each of the filters 93. That is, as illustrated in Fig. 11 as an example, pieces of the operation data 95 are generated for the number of filters 93 applied to the target data 92. In addition, the operation data 95 includes the plurality of elements 94 which are two-dimensionally arranged, and thus the operation data 95 has a width and a height. The number of pieces of the operation data 95 is called the number of channels. Fig. 11 illustrates four channels of pieces of the operation data 95 that are output by applying the four filters 93 to the target data 92.

As illustrated in Fig. 12 as an example, the compression unit 82 includes a pooling layer 100 represented by "pooling (abbreviated as pool)" in addition to the convolutional layer 90. The pooling layer 100 obtains local statistics of the element values of the elements 94 of the operation data 95, and generates reduction operation data 95S in which the obtained statistics are used as element values. Here, the pooling layer 100 performs maximum value pooling processing of obtaining, as the local statistic, a maximum value of the element values in a $2 \times 2$ element block 101. By performing the processing while shifting the block 101 by one element in a width direction and a height direction, a size of the reduction operation data 95S is reduced to 1/2 of a size of the original operation data 95. Fig. 12 illustrates a case where the element value b among the element values a, b, e, and f in the block 101A is a maximum value, the element value b among the element values b, c, f, and g in the block 101B is a maximum value, and the element value h among the element values c, d, g, and h in the block 101C is a maximum value. Average value pooling processing of obtaining, as a local statistic, an average value instead of the maximum value may be performed.

The compression unit 82 outputs final operation data 95 by repeating the convolution processing by the convolutional layer 90 and the pooling processing by the pooling layer 100 a plurality of times. The final operation data 95 is, in other words, the feature amount set 84, and the element value of each element 94 of the final operation data 95 is, in other words, the feature amount Z. The feature amount Z obtained in this way represents a shape of the anatomical region and a feature of a texture, such as a degree of atrophy of the hippocampus and the presence or absence of a decrease in blood flow metabolism of the frontotemporal lobe. Here, for the sake of simplicity, the description is given that the processing is performed in a two-dimensional manner. On the other hand, the processing is actually performed in a three-dimensional manner.

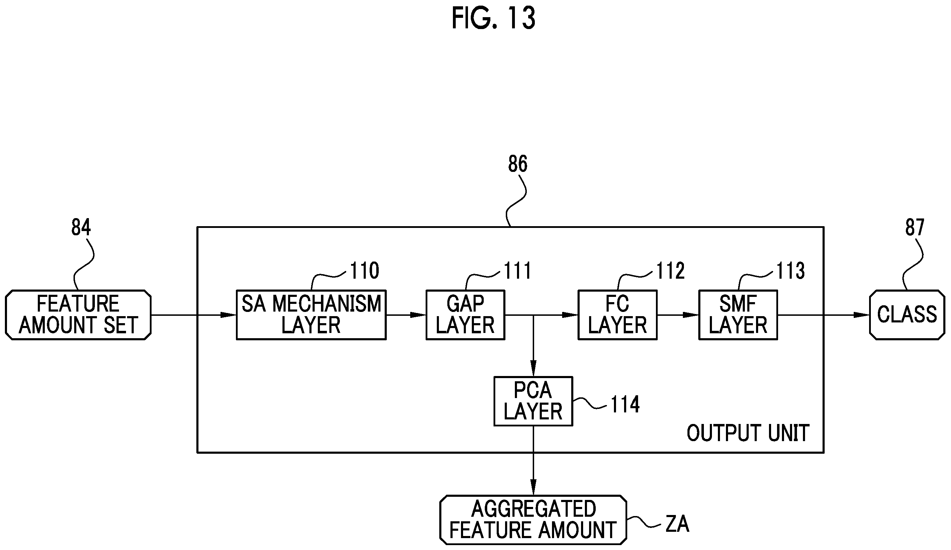

As illustrated in Fig. 13 as an example, the output unit 86 includes a self-attention (hereinafter, abbreviated as SA) mechanism layer 110, a global average pooling (hereinafter, abbreviated as GAP) layer 111, a fully connected (hereinafter, abbreviated as FC) layer 112, a softmax function (hereinafter, abbreviated as SMF) layer 113, and a principal component analysis (hereinafter, abbreviated as PCA) layer 114.

The SA mechanism layer 110 performs convolution processing illustrated in Fig. 10 on the feature amount set 84 while changing the coefficients of the filter 93 according to the element value of the element of interest 911. Hereinafter, the convolution processing performed by the SA mechanism layer 110 is referred to as SA convolution processing. The SA mechanism layer 110 outputs the feature amount set 84 after the SA convolution processing to the GAP layer 111.

The GAP layer 111 performs global average pooling processing on the feature amount set 84 after the SA convolution processing. The global average pooling processing is processing of obtaining average values of the feature amounts Z for each channel (refer to Fig. 11) of the feature amount set 84. For example, in a case where the number of channels of the feature amount set 84 is 512, average values of 512 feature amounts Z are obtained by the global average pooling processing. The GAP layer 111 outputs the obtained average values of the feature amounts Z to the FC layer 112 and the PCA layer 114.

The FC layer 112 converts the average values of the feature amounts Z into variables handled by the SMF of the SMF layer 113. The FC layer 112 includes an input layer including units corresponding to the number of the average values of the feature amounts Z (that is, the number of channels of the feature amount set 84) and an output layer including units corresponding to the number of variables handled by the SMF. Each unit of the input layer and each unit of the output layer are fully coupled to each other, and weights are set for each unit. The average values of the feature amounts Z are input to each unit of the input layer. The product sum of the average value of the feature amounts Z and the weight which is set for each unit is an output value of each unit of the output layer. The output value is the variable handled by the SMF. The FC layer 112 outputs the variables handled by the SMF to the SMF layer 113. The SMF layer 113 outputs the class 87 by applying the variables to the SMF.

The PCA layer 114 performs PCA on the average values of the feature amounts Z, and aggregates the average values of the plurality of feature amounts Z into aggregated feature amounts ZA of which the number is smaller than the number of the average values. For example, the PCA layer 114 aggregates the average values of 512 feature amounts Z into one aggregated feature amount ZA.

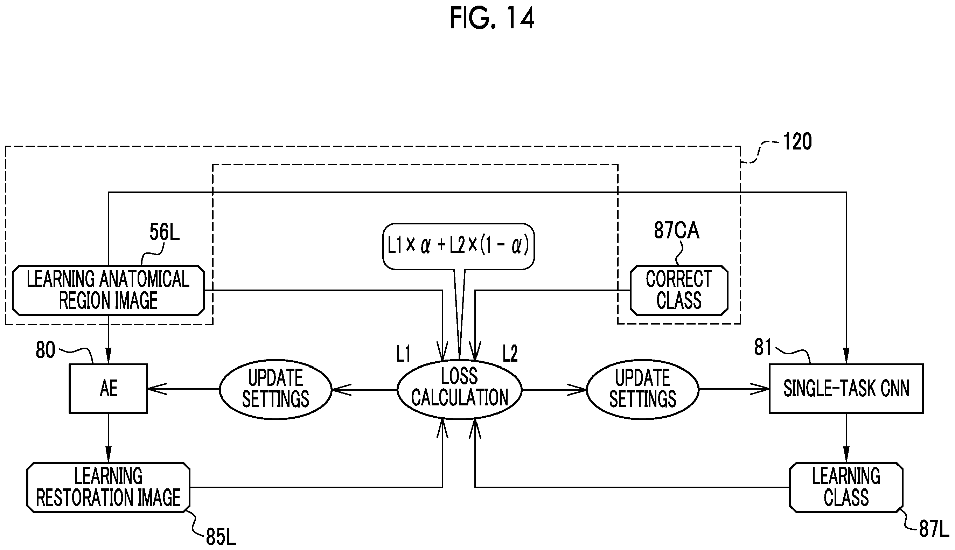

As illustrated in Fig. 14 as an example, the AE 80 is trained by inputting learning anatomical region images 56L in a learning phase. The AE 80 outputs learning restoration images 85L in response to the learning anatomical region images 56L. Loss calculation of the AE 80 using a loss function is performed based on the learning anatomical region images 56L and the learning restoration images 85L. In addition, update setting of various coefficients (coefficients of the filter 93 and the like) of the AE 80 is performed according to a result of the loss calculation (hereinafter, referred to as a loss L1), and the AE 80 is updated according to the update setting.

In the learning phase of the AE 80, while exchanging the learning anatomical region images 56L, a series of processing including inputting of the learning anatomical region images 56L to the AE 80, outputting of the learning restoration images 85L from the AE 80, the loss calculation, the update setting, and updating of the AE 80 is repeatedly performed.

The single-task CNN 81 is trained by inputting learning data 120 in a learning phase. The learning data 120 is a set of the learning anatomical region image 56L and a correct class 87CA corresponding to the learning anatomical region image 56L. The correct class 87CA indicates whether the patient P in the learning anatomical region image 56L is actually sMCI or cMCI.

In the learning phase, the learning anatomical region image 56L is input to the single-task CNN 81. The single-task CNN 81 outputs a learning class 87L in response to the learning anatomical region image 56L. The loss calculation of the single-task CNN 81 using a cross-entropy function or the like is performed based on the learning class 87L and the correct class 87CA. In addition, update setting of various coefficients of the single-task CNN 81 is performed according to a result of the loss calculation (hereinafter, referred to as a loss L2), and the single-task CNN 81 is updated according to the update setting.

In the learning phase of the single-task CNN 81, while exchanging the learning data 120, a series of processing including inputting of the learning anatomical region image 56L to the single-task CNN 81, outputting of the learning class 87L from the single-task CNN 81, the loss calculation, the update setting, and updating of the single-task CNN 81 is repeatedly performed.

The update setting of the AE 80 and the update setting of the single-task CNN 81 are performed based on a total loss L represented by the following equation (2). $\alpha$ is a weight.

$$L = L1 \times \alpha + L2 \times (1\text{-}\alpha) \cdots (2)$$

That is, the total loss L is a weighted sum of the loss L1 of the AE 80 and the loss L2 of the single-task CNN 81.

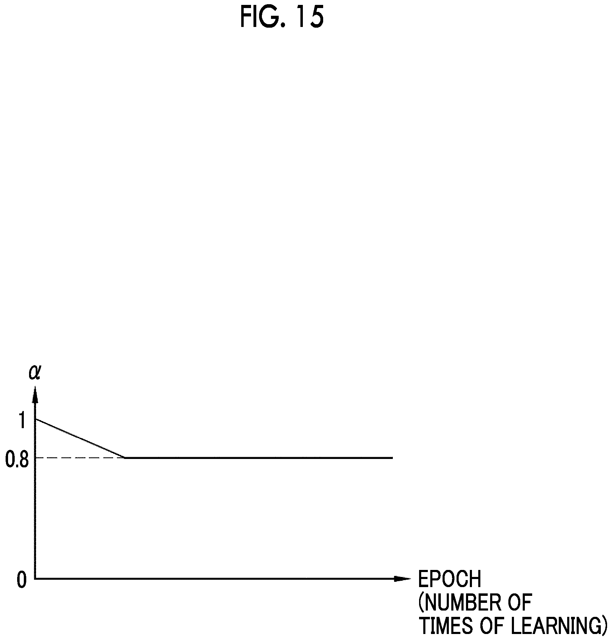

As illustrated in Fig. 15 as an example, the weight $\alpha$ is set to 1 in an initial stage of the learning phase. Assuming that the weight $\alpha$ is 1, the total loss L is represented by L = L1. Therefore, in this case, only the learning of the AE 80 is performed, and the learning of the single-task CNN 81 is not performed.

The weight $\alpha$ is gradually decreased from 1 as the learning is progressed, and is eventually set as a fixed value (0.8 in Fig. 15). In this case, the learning of the AE 80 and the learning of the single-task CNN 81 are both performed with intensity corresponding to the weight $\alpha$. As described above, the weight given to the loss L1 is larger than the weight given to the loss L2. Further, the weight given to the loss L1 is gradually decreased from a maximum value of 1, and the weight given to the loss L2 is gradually increased from a minimum value of 0. Both the weight given to the loss L1 and the weight given to the loss L2 are set as fixed values.

The learning of the AE 80 and the single-task CNN 81 is ended in a case where accuracy of restoration from the learning anatomical region image 56L to the learning restoration image 85L by the AE 80 reaches a predetermined setting level and where prediction accuracy of the learning class 87L with respect to the correct class 87CA by the single-task CNN 81 reaches a predetermined setting level. The AE 80 of which the restoration accuracy reaches the setting level in this way and the single-task CNN 81 of which the prediction accuracy reaches the setting level in this way are stored in the storage 20, and are used as the feature amount derivation model 37.

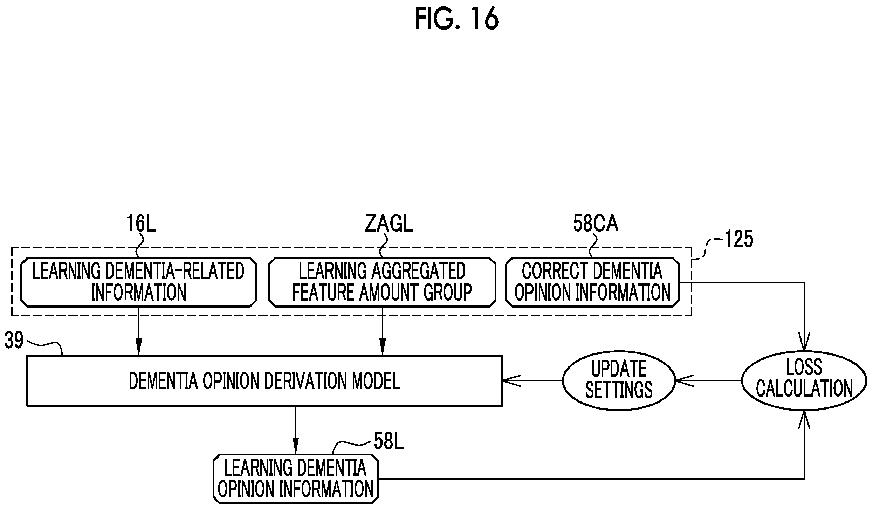

As illustrated in Fig. 16 as an example, in the learning phase, the dementia opinion derivation model 39 is trained by inputting learning data 125. The learning data 125 is a combination of learning dementia-related information 16L and learning aggregated feature amount group ZAGL, and correct dementia opinion information 58CA corresponding to the learning dementia-related information 16L and the learning aggregated feature amount group ZAGL. The learning aggregated feature amount group ZAGL is obtained by inputting the anatomical region image 56 of a certain head MRI image 15 to the feature amount derivation model 37. The learning dementia-related information 16L is information of the patient P whose the head MRI image 15 is imaged, the head MRI image 15 being an image from which the learning aggregated feature amount group ZAGL is obtained. The correct dementia opinion information 58CA is a result obtained by actually diagnosing, by the doctor, the dementia opinion on the head MRI image 15 from which the learning aggregated feature amount group ZAGL is obtained.

In the learning phase, the learning dementia-related information 16L and the learning aggregated feature amount group ZAGL are input to the dementia opinion derivation model 39. The dementia opinion derivation model 39 outputs the learning dementia opinion information 58L in response to the learning dementia-related information 16L and the learning aggregated feature amount group ZAGL. A loss calculation of the dementia opinion derivation model 39 using a loss function is performed based on the learning dementia opinion information 58L and the correct dementia opinion information 58CA. In addition, update setting of various coefficients of the dementia opinion derivation model 39 is performed according to a result of the loss calculation, and the dementia opinion derivation model 39 is updated according to the update setting.

In the learning phase of the dementia opinion derivation model 39, while exchanging the learning data 125, a series of processing including inputting of the learning dementia-related information 16L and the learning aggregated feature amount group ZAGL to the dementia opinion derivation model 39, outputting of the learning dementia opinion information 58L from the dementia opinion derivation model 39, the loss calculation, the update setting, and updating of the dementia opinion derivation model 39 is repeatedly performed. The repetition of the series of pieces of processing is ended in a case where prediction accuracy of the learning dementia opinion information 58L with respect to the correct dementia opinion information 58CA reaches a predetermined setting level. The dementia opinion derivation model 39 of which the prediction accuracy reaches the setting level in this way is stored in the storage 20, and is used in the dementia opinion derivation unit 49.

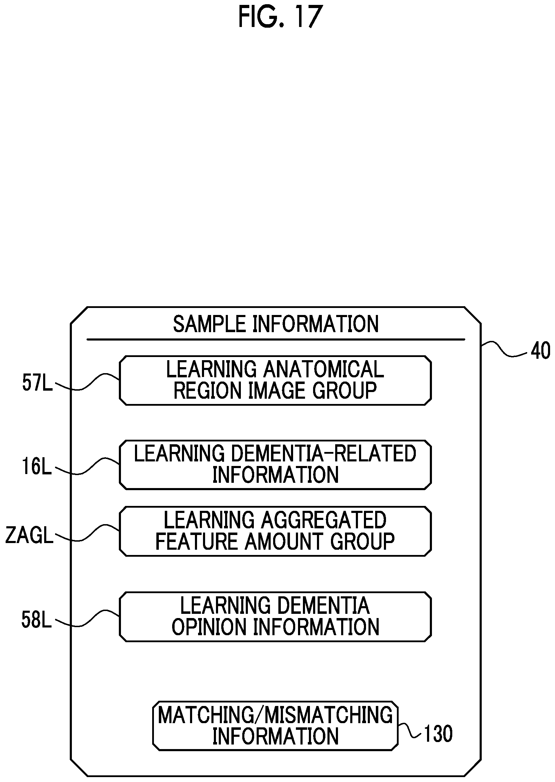

As illustrated in Fig. 17 as an example, the sample information 40 is information on a sample obtained by inputting pieces of input data to the feature amount derivation model 37 and the dementia opinion derivation model 39 in the learning phase. As illustrated in Fig. 14, the pieces of input data of the feature amount derivation model 37 in the learning phase are the learning anatomical region images 56L. In addition, as illustrated in Fig. 16, the pieces of input data of the dementia opinion derivation model 39 in the learning phase are the learning dementia-related information 16L and the learning aggregated feature amount group ZAGL. The sample information 40 includes each of the pieces of input data, that is, a learning anatomical region image group 57L which is a set of the learning anatomical region images 56L, the learning dementia-related information 16L, and the learning aggregated feature amount group ZAGL.

In addition, the sample information 40 includes the learning dementia opinion information 58L and matching/mismatching information 130. The matching/mismatching information 130 is information indicating matching/mismatching of the prediction of the dementia opinion by the dementia opinion derivation model 39. Specifically, the matching/mismatching information 130 is information indicating matching/mismatching between the learning dementia opinion information 58L and the correct dementia opinion information 58CA which is an actual result.

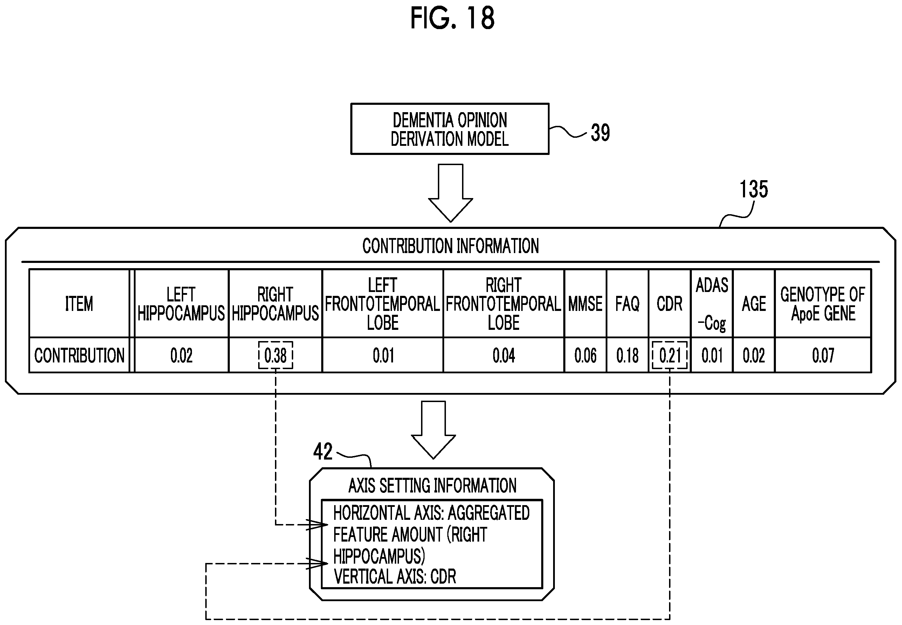

As illustrated in Fig. 18 as an example, since the dementia opinion derivation model 39 is constructed by a linear discriminant analysis method, contribution information 135 can be derived. The contribution information 135 is information in which a contribution of each item of the learning dementia-related information 16L and the learning aggregated feature amount group ZAGL to the learning dementia opinion information 58L is registered. The contribution has a larger value as the item largely contributes to the derivation of the learning dementia opinion information 58L.

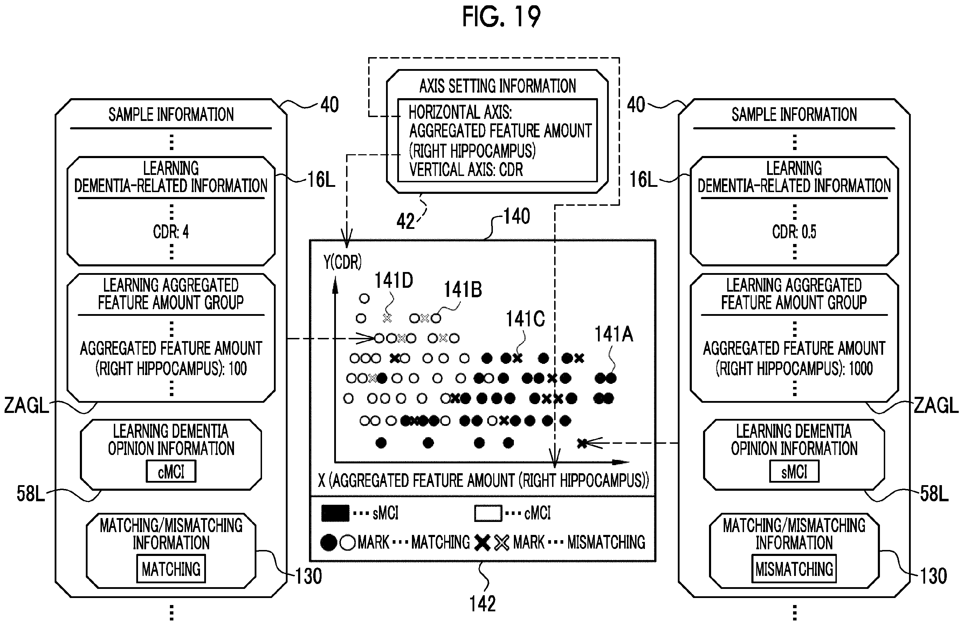

The axis setting information 42 is information for setting a horizontal axis and a vertical axis of a scatter diagram 140 (refer to Fig. 19 and the like) to be described later. The axis setting information 42 is generated based on the contribution information 135. That is, among the plurality of pieces of input data of the dementia opinion derivation model 39, such as the aggregated feature amount ZA_1 of the left hippocampus, the aggregated feature amount ZA_4 of the right frontotemporal lobe, the MMSE score, and the age, parameters related to the pieces of input data having a first contribution and a second contribution are set as the horizontal axis and the vertical axis.

Fig. 18 illustrates a case where the aggregated feature amount ZA_2 of the right hippocampus has a first contribution of 0.38 and the CDR has a second contribution of 0.21. In this case, the aggregation feature amount ZA_2 of the right hippocampus is set as the horizontal axis, and the CDR is set as the vertical axis. The aggregated feature amount ZA_2 of the right hippocampus and the CDR are an example of "parameters" according to the technique of the present disclosure.

As illustrated in Fig. 19 as an example, the display control unit 50 generates the scatter diagram 140 with reference to the sample information 40 and the axis setting information 42. In the scatter diagram 140, marks 141 representing a plurality of samples are plotted in a two-dimensional space in which two parameters are set as the horizontal axis and the vertical axis, the two parameters being set based on a plurality of types of input data of the dementia opinion derivation model 39. As in the case of Fig. 18, Fig. 19 illustrates a case where the aggregated feature amount ZA_2 of the right hippocampus is set as the horizontal axis and the CDR is set as the vertical axis.

There are four types of marks 141 including marks 141A, 141B, 141C, and 141D. As illustrated in exemplification 142, the mark 141A is, for example, a circle mark filled in blue. The mark 141A is assigned to a sample in which the learning dementia opinion information 58L is sMCI and the matching/mismatching information 130 indicates matching. The mark 141B is, for example, a circle mark filled in red. The mark 141B is assigned to a sample in which the learning dementia opinion information 58L is cMCI and the matching/mismatching information 130 indicates matching.

The mark 141C is, for example, a cross mark filled in blue. The mark 141C is assigned to a sample in which the learning dementia opinion information 58L is sMCI and the matching/mismatching information 130 indicates mismatching. The mark 141D is, for example, a cross mark filled in red. The mark 141D is assigned to a sample in which the learning dementia opinion information 58L is cMCI and the matching/mismatching information 130 indicates mismatching. As described above, the mark 141 indicates whether the learning dementia opinion information 58L is sMCI or cMCI, that is, a type of the output data. In addition, the mark 141 indicates matching/mismatching between the learning dementia opinion information 58L and the correct dementia opinion information 58CA, that is, matching/mismatching between the output data and the actual result.

Fig. 19 illustrates a state where the mark 141B which is a circle mark filled in red is assigned to the sample in which the CDR of the learning dementia-related information 16L is 4, the aggregated feature amount ZA_2 of the right hippocampus included in the learning aggregated feature amount group ZAGL is 100, the learning dementia opinion information 58L is cMCI, and the matching/mismatching information 130 is matching. In addition, Fig. 19 illustrates a state where the mark 141C which is a cross mark filled in blue is assigned to the sample in which the CDR of the learning dementia-related information 16L is 0.5, the aggregated feature amount ZA_2 of the right hippocampus included in the learning aggregated feature amount group ZAGL is 1000, the learning dementia opinion information 58L is sMCI, and the matching/mismatching information 130 is mismatching.

Fig. 20 illustrates an example of the first display screen 150 for instructing the analysis by the segmentation model 36, the feature amount derivation model 37, and the dementia opinion derivation model 39. The head MRI images 15 of the patient P for diagnosing dementia are displayed on the first display screen 150. The head MRI images 15 include a head MRI image 15S having a sagittal cross section, a head MRI image 15A having an axial cross section, and a head MRI image 15C having a coronal cross section. A button group 151 for switching the display is provided in a lower portion of each of the head MRI images 15S, 15A, and 15C.

An analysis button 152 is provided on the first display screen 150. The doctor selects the analysis button 152 in a case where he/she wants to perform analysis using the segmentation model 36, the feature amount derivation model 37, and the dementia opinion derivation model 39. In response to the selection, the CPU 22 receives an instruction for analysis by the segmentation model 36, the feature amount derivation model 37, and the dementia opinion derivation model 39.

Fig. 21 illustrates an example of a second display screen 155 for displaying the dementia opinion information 58 obtained as a result of analysis by the segmentation model 36, the feature amount derivation model 37, and the dementia opinion derivation model 39. On the second display screen 155, a message 156 according to the dementia opinion information 58 is displayed. Fig. 21 illustrates an example in which the dementia opinion information 58 includes content of cMCI and a message "There is a possibility of progressing to Alzheimer's disease after two years" is displayed as the message 156.

A confirmation button 157 and a verification button 158 are provided in a lower portion of the second display screen 155. In a case where the confirmation button 157 is selected, the display control unit 50 turns off the display of the message 156, and returns the second display screen 155 to the first display screen 150. In addition, in a case where the verification button 158 is selected, the display control unit 50 displays a verification screen 160 illustrated in Fig. 22 on the display 17.

As illustrated in Fig. 22 as an example, on the verification screen 160, the contribution information 135, the scatter diagram 140, and the exemplification 142 are displayed. A mark 161 representing a target sample is displayed on the scatter diagram 140. The mark 161 is, for example, a rhombic mark filled in black. The target sample is a sample to be analyzed by the segmentation model 36, the feature amount derivation model 37, and the dementia opinion derivation model 39, and is a sample for which the dementia opinion information 58 is displayed on the second display screen 155 illustrated in Fig. 21.

A target sample information display region 162 for displaying various types of information of the target sample is displayed on a left side of the scatter diagram 140. The target sample information display region 162 is divided into an anatomical region image display region 163, a dementia-related information display region 164, and a dementia opinion information display region 165. In the anatomical region image display region 163, for the target sample, the anatomical region image 56_1 of the left hippocampus, the anatomical region image 56_2 of the right hippocampus, the anatomical region image 56_3 of the left frontotemporal lobe, and the anatomical region image 56_4 of the right frontotemporal lobe are displayed. In the dementia-related information display region 164, the dementia-related information 16 of the target sample is displayed. In the dementia opinion information display region 165, the dementia opinion information 58 of the target sample is displayed. In the target sample information display region 162, a frame 166 surrounding the pieces of input data which are set as the horizontal axis and the vertical axis of the scatter diagram 140 (in this example, the anatomical region image 56_2 of the right hippocampus based on the aggregated feature amount ZA_2 of the right hippocampus, and the CDR) is displayed. The display control unit 50 turns off the display of the verification screen 160 in a case where a close button 167 is selected.

The mark 141 of the scatter diagram 140 can be selected by a cursor 168 operated via the input device 18. The doctor places the cursor 168 on the mark 141 of a sample (hereinafter, referred to as a comparison sample) to be compared with the target sample and selects the sample.

As illustrated in Fig. 23 as an example, in a case where the mark 141 is selected, a comparison sample information display region 170 for displaying various types of information of the comparison sample corresponding to the selected mark 141 is displayed on a right side of the scatter diagram 140. The comparison sample information display region 170 is divided into a learning anatomical region image display region 171, a learning dementia-related information display region 172, a learning dementia opinion information display region 173, and a matching/mismatching information display region 174. In the learning anatomical region image display region 171, for the comparison sample, a learning anatomical region image 56_1L of a left hippocampus, a learning anatomical region image 56_2L of a right hippocampus, a learning anatomical region image 56_3L of a left frontotemporal lobe, and a learning anatomical region image 56_4L of a right frontotemporal lobe are displayed. In the learning dementia-related information display region 172, learning dementia-related information 16L of the comparison sample is displayed. In the learning dementia opinion information display region 173, learning dementia opinion information 58L of the comparison sample is displayed. In the matching/mismatching information display region 174, matching/mismatching information 130 of the comparison sample is displayed. The display content of the comparison sample information display region 170 is switched to information of the comparison sample corresponding to the selected mark 141 each time another mark 141 is selected. Similarly to the target sample information display region 162, a frame 166 is also displayed in the comparison sample information display region 170.

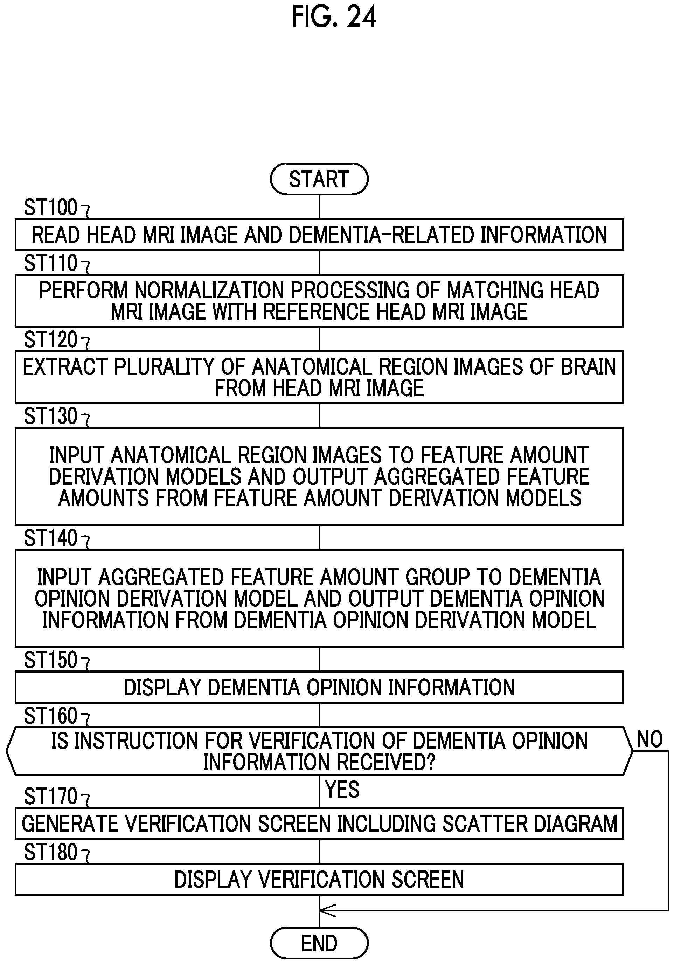

Next, an operation according to the configuration will be described with reference to a flowchart illustrated in Fig. 24. First, in a case where the operation program 30 is started in the diagnosis support device 13, as illustrated in Fig. 4, the CPU 22 of the diagnosis support device 13 functions as the RW control unit 45, the normalization unit 46, the extraction unit 47, the feature amount derivation unit 48, the dementia opinion derivation unit 49, and the display control unit 50.

In a case where the analysis button 152 is selected on the first display screen 150 illustrated in Fig. 20, the RW control unit 45 reads the corresponding head MRI image 15 and the corresponding dementia-related information 16, and the reference head MRI image 35 from the storage 20 (step ST100). The head MRI image 15 and the reference head MRI image 35 are output from the RW control unit 45 to the normalization unit 46. The dementia-related information 16 is output from the RW control unit 45 to the dementia opinion derivation unit 49.

As illustrated in Fig. 5, the normalization unit 46 performs normalization processing (shape normalization processing 65 and shade normalization processing 66) of matching the head MRI image 15 with the reference head MRI image 35 (step ST110). Thereby, the head MRI image 15 is set as a normalized head MRI image 55. The normalized head MRI image 55 is output from the normalization unit 46 to the extraction unit 47.

As illustrated in Fig. 6, the extraction unit 47 extracts a plurality of anatomical region images 56 of the brain from the normalized head MRI image 55 using the segmentation model 36 (step ST120). The anatomical region image group 57 including the plurality of anatomical region images 56 is output from the extraction unit 47 to the feature amount derivation unit 48.

As illustrated in Fig. 7, the feature amount derivation unit 48 inputs the anatomical region images 56 to the corresponding feature amount derivation models 37. Thereby, the aggregated feature amounts ZA are output from the feature amount derivation models 37 (step ST130). The aggregated feature amount group ZAG including the plurality of aggregated feature amounts ZA is output from the feature amount derivation unit 48 to the dementia opinion derivation unit 49.

As illustrated in Fig. 8, the dementia opinion derivation unit 49 inputs the dementia-related information 16 and the aggregated feature amount group ZAG to the dementia opinion derivation model 39. Thereby, the dementia opinion information 58 is output from the dementia opinion derivation model 39 (step ST140). The dementia opinion information 58 is output from the dementia opinion derivation unit 49 to the display control unit 50.

Under a control of the display control unit 50, the second display screen 155 illustrated in Fig. 21 is displayed on the display 17 (step ST150). A doctor confirms the dementia opinion information 58 via the message 156 on the second display screen 155.

In a case where the doctor desires to verify the validity of the dementia opinion information 58, the doctor selects the verification button 158 of the second display screen 155. Thereby, an instruction for verification of the dementia opinion information 58 is received by the CPU 22 (YES in step ST160). In this case, as illustrated in Fig. 19, the display control unit 50 generates the verification screen 160 including the scatter diagram 140 illustrated in Fig. 22 and Fig. 23 (step ST170). In addition, the verification screen 160 is displayed on the display 17 under the control of the display control unit 50 (step ST180). The doctor verifies the validity of the dementia opinion information 58 of the target sample via the target sample information display region 162 and the comparison sample information display region 170 of the verification screen 160.

As described above, the CPU 22 of the diagnosis support device 13 includes the display control unit 50. The display control unit 50 generates the scatter diagram 140 for the dementia opinion derivation model 39 that receives the plurality of types of input data such as the dementia-related information 16 and the aggregated feature amount group ZAG and outputs the dementia opinion information 58 which is the output data according to the input data. The scatter diagram 140 is obtained by plotting the marks 141 representing the plurality of samples in a two-dimensional space in which two parameters are set as a horizontal axis and a vertical axis, the samples being obtained by inputting the pieces of input data to the dementia opinion derivation model 39, and the two parameters being set based on the plurality of types of input data. The display control unit 50 displays the scatter diagram 140, the input data, and the type of the output data on the display 17. Therefore, even in the multimodal learning in which a plurality of types of data are used as input data, it is possible to easily verify the validity of the dementia opinion information 58.

The display control unit 50 displays the scatter diagram 140 in a form in which the marks 141 can be selected. In a case where the mark 141 is selected, the display control unit 50 displays at least the input data of the sample corresponding to the selected mark 141. Therefore, the input data can be displayed by a simple operation of selecting the mark 141. In addition, the sample represented by the mark 141 in which a distance from the mark 161 of the target sample is relatively short is a sample similar to the target sample. Therefore, in a case where the mark 141 in which the distance from the mark 161 of the target sample is relatively short is selected, it is possible to compare the target sample with a comparison sample similar to the target sample, and more easily verify the validity of the dementia opinion information 58.

As illustrated in Fig. 23, the display control unit 50 displays pieces of input data and types of pieces of output data of two samples in a comparable manner. Therefore, it is possible to easily compare the target sample and the comparison sample, and to verify the validity of the dementia opinion information 58. The pieces of input data and the types of pieces of output data of three or more samples may be displayed in a comparable manner.

As illustrated in Fig. 19 and the like, the mark 141 represents the type of the output data. Therefore, only by viewing the scatter diagram 140 at a glance, it is possible to recognize a tendency of the types of pieces of output data with respect to two pieces of input data which are set as the horizontal axis and the vertical axis. For example, in the scatter diagram 140 illustrated in Fig. 19 and the like, it can be seen that the dementia opinion information 58 tends to be cMCI as the aggregated feature amount ZA_2 of the hippocampus is lower and the CDR is higher. In addition, on the contrary, it can be seen that the dementia opinion information 58 tends to be sMCI as the aggregated feature amount ZA_2 of the hippocampus is higher and the CDR is lower.

Further, the mark 141 represents matching/mismatching between the output data and the actual result. Therefore, only by viewing the scatter diagram 140 at a glance, it is possible to recognize matching/mismatching between the output data of each sample and the actual result.

The display control unit 50 sets, as the horizontal axis and the vertical axis of the scatter diagram 140, two related parameters which are preset in the axis setting information 42 among the plurality of types of input data. Therefore, the doctor does not need to take a time and effort to set the horizontal axis and the vertical axis.

As illustrated in Fig. 8, the dementia opinion derivation model 39 is constructed by a method capable of deriving the contribution of each of the plurality of types of input data to the output data, that is, linear discriminant analysis. As illustrated in Fig. 18 and Fig. 19, the display control unit 50 sets, as the horizontal axis and the vertical axis of the scatter diagram 140, parameters related to the pieces of input data which have a first contribution and a second contribution among the plurality of types of input data. Therefore, it is possible to generate the scatter diagram 140 in which the tendency of the types of pieces of output data can be more easily recognized.

As illustrated in Fig. 7 and Fig. 8, the plurality of types of input data include the aggregated feature amounts ZA, which are obtained by inputting the anatomical region images 56 of the plurality of anatomical regions extracted from the head MRI image 15 (normalized head MRI image 55) to the feature amount derivation models 37 prepared corresponding to the plurality of anatomical regions, respectively. The aggregated feature amounts ZA represent comprehensive features of the brain. In addition, the aggregated feature amount ZA is obtained by inputting the anatomical region image 56 to the feature amount derivation model 37. Therefore, it is possible to improve the prediction accuracy of the dementia opinion by the dementia opinion derivation model 39.

In dementia, as compared with other diseases such as cancer, specific lesions that can be recognized with the naked eye are less likely to appear in the image. In addition, dementia has an effect on the entire brain, and is not local. Because of this background, in the related art, it is difficult to obtain an accurate dementia opinion from a medical image such as a head MRI image 15 by using a machine learning model. On the other hand, according to the technique of the present disclosure, the brain is subdivided into the plurality of anatomical regions, the plurality of anatomical region images 56 are generated from the plurality of anatomical regions, and the aggregated feature amounts ZA are derived for each of the plurality of anatomical region images 56. In addition, the plurality of aggregated feature amounts ZA which are derived are input to one dementia opinion derivation model 39. Therefore, it is possible to achieve the object for obtaining a more accurate dementia opinion, as compared with the technique in the related art in which it is difficult to obtain an accurate dementia opinion.

In addition, as illustrated in Fig. 8, the plurality of types of input data include the dementia-related information 16 related to dementia. Pieces of powerful information useful for prediction of a dementia opinion such as the dementia-related information 16 are added. Thus, as compared with the case where the dementia opinion is predicted by using only the aggregated feature amount group ZAG, it is possible to dramatically improve the prediction accuracy of the dementia opinion. The dementia-related information 16 may not be included as the input data.

As illustrated in Fig. 9, the feature amount derivation model 37 is obtained by adapting a model in which the AE 80 and the single-task CNN 81 are combined. The AE 80 and the single-task CNN 81 are also one of neural network models which are frequently used in the field of machine learning, and are generally very well known. Therefore, the AE 80 and the single-task CNN 81 can be relatively easily adapted as the feature amount derivation model 37.

The single-task CNN 81 that performs a main task such as outputting of the class 87 and the AE 80 that is partially common to the single-task CNN 81 and performs a sub-task such as generation of the restoration image 85 are used as the feature amount derivation model 37, the sub-task being a task having a more general purpose as compared with the main task. In addition, the AE 80 and the single-task CNN 81 are trained at the same time. Therefore, as compared with a case where the AE 80 and the single-task CNN 81 are separate, the feature amount set 84 that is more appropriate and the aggregated feature amounts ZA that are more appropriate can be output. As a result, it is possible to improve the prediction accuracy of the dementia opinion information 58.

In the learning phase, the update setting is performed based on the total loss L, which is a weighted sum of the loss L1 of the AE 80 and the loss L2 of the single-task CNN 81. Therefore, by setting the weight $\alpha$ to an appropriate value, the AE 80 can be intensively trained, the single-task CNN 81 can be intensively trained, or the AE 80 and the single-task CNN 81 can be trained in a well-balanced manner.

The weight given to the loss L1 is larger than the weight given to the loss L2. Therefore, the AE 80 can always be intensively trained. In a case where the AE 80 is always intensively trained, the feature amount set 84 that more represents the shape of the anatomical region and the feature of the texture can be output from the compression unit 82. As a result, the aggregated feature amounts ZA having a higher plausibility can be output from the output unit 86.

Further, the weight given to the loss L1 is gradually decreased from the maximum value, and the weight given to the loss L2 is gradually increased from the minimum value. After the learning is performed a predetermined number of times, both the weight given to the loss L1 and the weight given to the loss L2 are set as fixed values. Thus, the AE 80 can be more intensively trained in an initial stage of the learning. The AE 80 is responsible for a relatively simple sub-task such as generation of the restoration image 85. Therefore, in a case where the AE 80 is more intensively trained in the initial stage of the learning, the feature amount set 84 that more represents the shape of the anatomical region and the feature of the texture can be output from the compression unit 82 in the initial stage of the learning.

The dementia has become a social problem with the advent of an aging society in recent years. Therefore, it can be said that the present embodiment of outputting the dementia opinion information 58 in which a brain is set as an organ and dementia is set as a disease is a form that matches the current social problem.

The hippocampus and the frontotemporal lobe are anatomical regions that are particularly highly correlated with dementia such as Alzheimer's disease. Therefore, in a case where the plurality of anatomical regions include at least one of the hippocampus or the frontotemporal lobe, it is possible to obtain a more accurate dementia opinion.

In a case where the mark 141 represents the type of the output data, the dementia opinion information display region 165 and the learning dementia opinion information display region 173 may not be provided in the target sample information display region 162 and the comparison sample information display region 170. Similarly, in a case where the mark 141 represents matching/mismatching between the output data and the actual result, the matching/mismatching information display region 174 may not be provided in the comparison sample information display region 170.

The presentation form of the dementia opinion information 58 is not limited to the second display screen 155. The dementia opinion information 58 may be printed out on a paper medium, or the dementia opinion information 58 may be transmitted to a mobile terminal of the doctor as an attachment file of an e-mail.

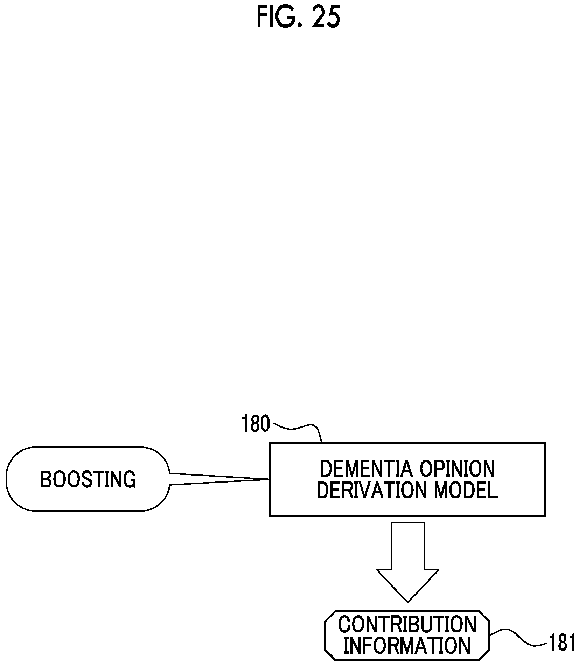

As illustrated in Fig. 25 as an example, a dementia opinion derivation model 180 constructed by a boosting method such as XGBoost instead of the linear discriminant analysis may be used. The dementia opinion derivation model 180 can derive contribution information 181 in the same manner as the dementia opinion derivation model 39. Although not illustrated, a dementia opinion derivation model constructed by a method using a neural network or a support vector machine may be used.

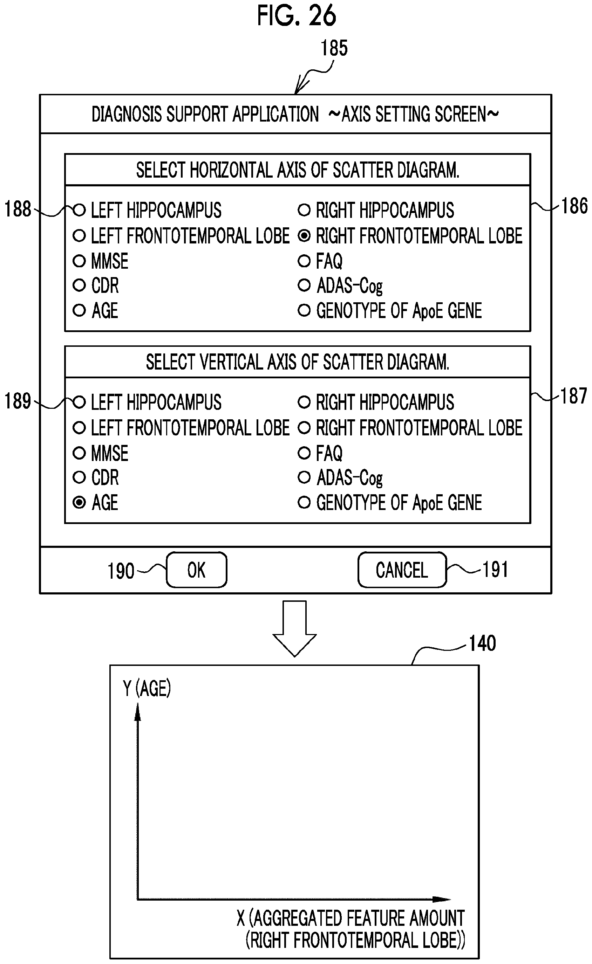

The horizontal axis and the vertical axis of the scatter diagram 140 are not limited to the parameters related to the pieces of input data having, for example, a first contribution and a second contribution. The parameters may be parameters related to two pieces of input data which are arbitrarily set. Alternatively, as illustrated in Fig. 26 as an example, two related parameters which are designated by the doctor may be set as the horizontal axis and the vertical axis of the scatter diagram 140.

In Fig. 26, for example, the display control unit 50 displays an axis designation screen 185 on the display 17 in a case where the verification button 158 of the second display screen 155 is selected. The axis designation screen 185 includes a horizontal axis designation region 186 and a vertical axis designation region 187. The horizontal axis designation region 186 is provided with a radio button 188 for alternatively selecting the plurality of types of input data such as the aggregated feature amount ZA_1 of the left hippocampus, the MMSE score, the FAQ, and the age. Similarly, the vertical axis designation region 187 is also provided with a radio button 189 for alternatively selecting the plurality of types of input data.

The doctor selects the radio buttons 188 and 189 of the pieces of input data to be designated as the horizontal axis and the vertical axis of the scatter diagram 140, and then selects an OK button 190. In a case where the OK button 190 is selected, the CPU 22 receives an instruction to designate the horizontal axis and the vertical axis of the scatter diagram 140. The display control unit 50 generates the scatter diagram 140 based on the horizontal axis and the vertical axis designated on the axis designation screen 185. Fig. 26 illustrates a case where the aggregated feature amount ZA_4 of the right frontotemporal lobe is designated as the horizontal axis and the age is designated as the vertical axis. In this case, the aggregated feature amount ZA_4 of the right frontotemporal lobe and the age are an example of "parameters" according to the technique of the present disclosure. In a case where a cancel button 191 is selected, the display control unit 50 turns off the display of the axis designation screen 185.

As described above, the display control unit 50 may set, as the horizontal axis and the vertical axis of the scatter diagram 140, the two related parameters which are designated by the doctor among the plurality of types of input data. It is possible to generate the scatter diagram 140 in which an intention of the doctor is reflected.

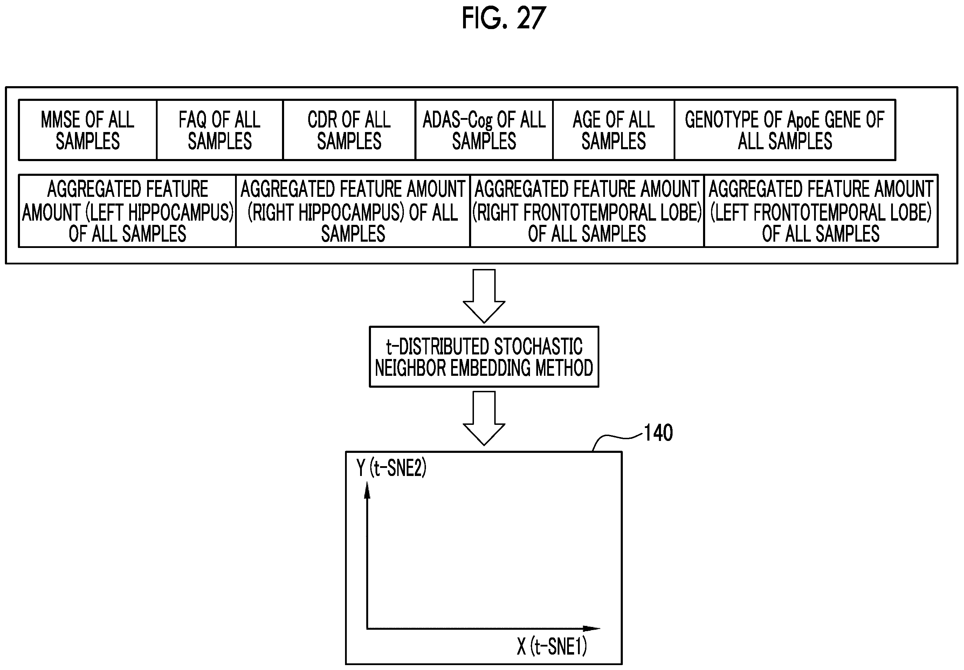

Alternatively, as illustrated in Fig. 27 as an example, the scatter diagram 140 may be generated by using a t-distributed stochastic neighbor embedding method (t-SNE). The t-distributed stochastic neighbor embedding method is, for example, a method often used for gene analysis, and in short, is a method of visualizing high-dimensional data by reducing the high-dimensional data to two-dimensional data or three-dimensional data. The t-distributed stochastic neighbor embedding method is described in, for example, the following literature.
<Laurens van der Maaten, etc., Visualizing data using t-SNE, Journal of Machine Learning Research, November 2008.>

In Fig. 27, in the form, the dementia-related information 16 of all samples such as the MMSE scores of all samples and the aggregated feature amount group ZAG of all samples such as the aggregated feature amount ZA_1 of the left hippocampus of all samples are analyzed by the t-distributed stochastic neighbor embedding method. In addition, the scatter diagram 140 in which t-SNE1 is set as the horizontal axis and t-SNE2 is set as the vertical axis is generated. t-SNE1 and t-SNE2 are an example of "parameters" according to the technique of the present disclosure. Even in such a method, the scatter diagram 140 can be generated without bothering the doctor.

A form of setting, as the horizontal axis and the vertical axis of the scatter diagram 140, parameters related to two pieces of input data which are preset, a form of setting, as the horizontal axis and the vertical axis of the scatter diagram 140, parameters related to two pieces of input data which are designated by the user, and a form of generating the scatter diagram by using the t-distributed stochastic neighbor embedding method may be configured to be selectable by the doctor.

[Second Embodiment]

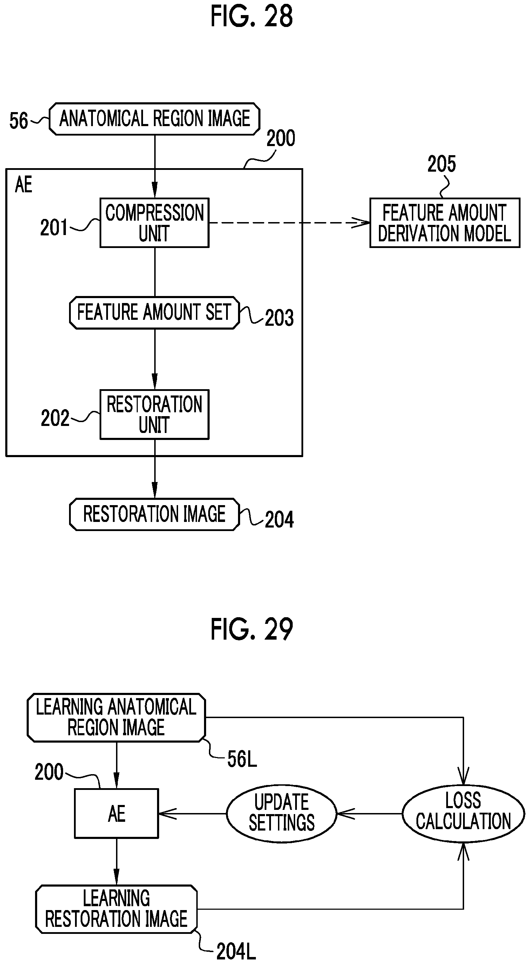

In a second embodiment illustrated in Fig. 28 to Fig. 30, a compression unit 201 of an AE 200 is used as a feature amount derivation model 205.

As illustrated in Fig. 28 as an example, the AE 200 includes a compression unit 201 and a restoration unit 202, similar to the AE 80 according to the first embodiment. The anatomical region image 56 is input to the compression unit 201. The compression unit 201 converts the anatomical region image 56 into the feature amount set 203. The compression unit 201 transmits the feature amount set 203 to the restoration unit 202. The restoration unit 202 generates a restoration image 204 of the anatomical region image 56 from the feature amount set 203.

As illustrated in Fig. 29 as an example, the AE 200 is trained by inputting learning anatomical region images 56L in a learning phase before the compression unit 201 is adapted as the feature amount derivation model 205. The AE 200 outputs learning restoration images 204L in response to the learning anatomical region images 56L. Loss calculation of the AE 200 using a loss function is performed based on the learning anatomical region images 56L and the learning restoration images 204L. In addition, update setting of various coefficients of the AE 200 is performed according to a result of the loss calculation, and the AE 200 is updated according to the update setting.

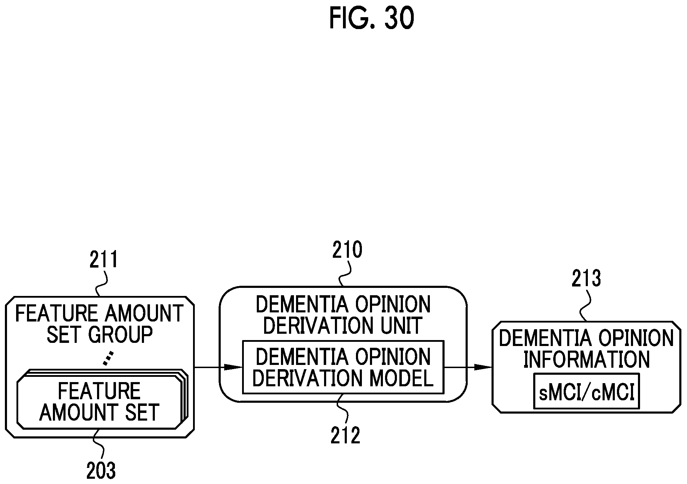

In the learning phase of the AE 200, while exchanging the learning anatomical region images 56L, a series of processing including inputting of the learning anatomical region images 56L to the AE 200, outputting of the learning restoration images 204L from the AE 200, the loss calculation, the update setting, and updating of the AE 200 is repeatedly performed. The repetition of the series of processing is ended in a case where accuracy of restoration from the learning anatomical region images 56L to the learning restoration images 204L reaches a predetermined setting level. The compression unit 201 of the AE 200 of which the restoration accuracy reaches the setting level in this manner is used as the feature amount derivation model 205 by being stored in the storage 20. Therefore, in the present embodiment, the feature amount set 203 which is output from the compression unit 201 is treated as "feature amount data" according to the technique of the present disclosure (refer to Fig. 30).

As illustrated in Fig. 30 as an example, the dementia opinion derivation unit 210 according to the present embodiment inputs a feature amount set group 211 to a dementia opinion derivation model 212. In addition, dementia opinion information 213 is output from the dementia opinion derivation model 212. The feature amount set group 211 includes a plurality of feature amount sets 203 which are output from the feature amount derivation model 205 for each of the plurality of anatomical region images 56. The dementia opinion information 213 has the same contents as the dementia opinion information 58 according to the first embodiment.

In this way, in the second embodiment, the compression unit 201 of the AE 200 is used as the feature amount derivation model 205. As described above, the AE 200 is one of neural network models which are frequently used in the field of machine learning, and thus the AE 200 can be relatively easily adapted as the feature amount derivation model 205.

[Third Embodiment]

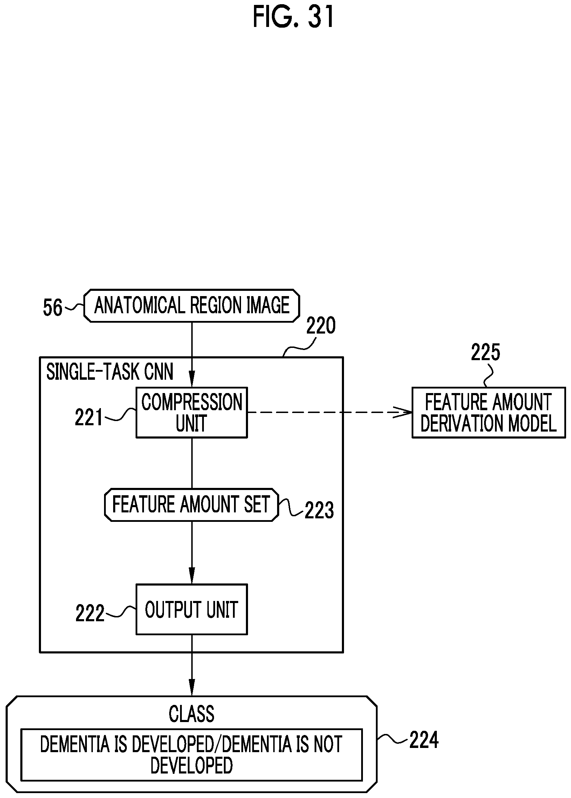

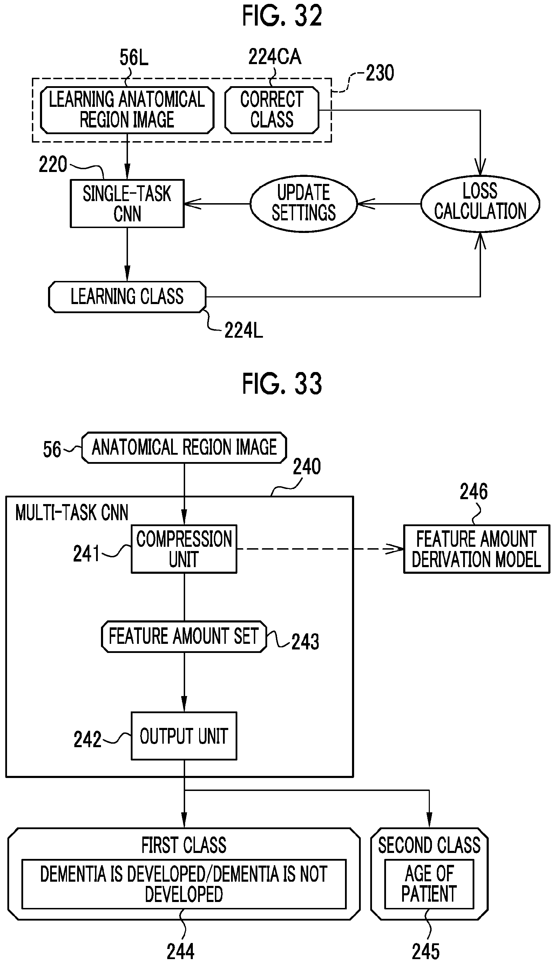

In a third embodiment illustrated in Fig. 31 and Fig. 32, a compression unit 221 of a single-task CNN 220 is used as the feature amount derivation model 225.

As illustrated in Fig. 31 as an example, the single-task CNN 220 includes a compression unit 221 and an output unit 222, similar to the single-task CNN 81 according to the first embodiment. The anatomical region image 56 is input to the compression unit 221. The compression unit 221 converts the anatomical region image 56 into the feature amount set 223. The compression unit 221 transmits the feature amount set 223 to the output unit 222. The output unit 222 outputs one class 224 based on the feature amount set 223. In Fig. 27, the output unit 222 outputs, as the class 224, a determination result indicating whether dementia is developed or not developed.

As illustrated in Fig. 32 as an example, the single-task CNN 220 is trained by inputting learning data 230 in a learning phase before the compression unit 221 is adapted as the feature amount derivation model 225. The learning data 230 is a set of the learning anatomical region image 56L and a correct class 224CA corresponding to the learning anatomical region image 56L. The correct class 224CA is a result obtained by actually determining, by the doctor, whether or not dementia is developed on the learning anatomical region image 56L.

In the learning phase, the learning anatomical region image 56L is input to the single-task CNN 220. The single-task CNN 220 outputs a learning class 224L in response to the learning anatomical region image 56L. The loss calculation of the single-task CNN 220 is performed based on the learning class 224L and the correct class 224CA. In addition, update setting of various coefficients of the single-task CNN 220 is performed according to a result of the loss calculation, and the single-task CNN 220 is updated according to the update setting.

In the learning phase of the single-task CNN 220, while exchanging the learning data 230, a series of processing including inputting of the learning anatomical region image 56L to the single-task CNN 220, outputting of the learning class 224L from the single-task CNN 220, the loss calculation, the update setting, and updating of the single-task CNN 220 is repeatedly performed. The repetition of the series of processing is ended in a case where prediction accuracy of the learning class 224L with respect to the correct class 224CA reaches a predetermined setting level. The compression unit 221 of the single-task CNN 220 of which the prediction accuracy reaches the setting level is stored in the storage 20, and is used as the feature amount derivation model 225. Similarly to the second embodiment, even in the present embodiment, the feature amount set 223 which is output from the compression unit 221 is treated as "feature amount data" according to the technique of the present disclosure.

As described above, in the third embodiment, the compression unit 221 of the single-task CNN 220 is used as the feature amount derivation model 225. As described above, the single-task CNN 220 is also one of neural network models which are frequently used in the field of machine learning, and thus the single-task CNN 220 can be relatively easily adapted as the feature amount derivation model 225.

The class 224 may include, for example, content indicating that the patient P is younger than 75 years old or content indicating that the patient P is 75 years old or older, or may include an age group of the patient P such as 60's and 70's.

[Fourth Embodiment]

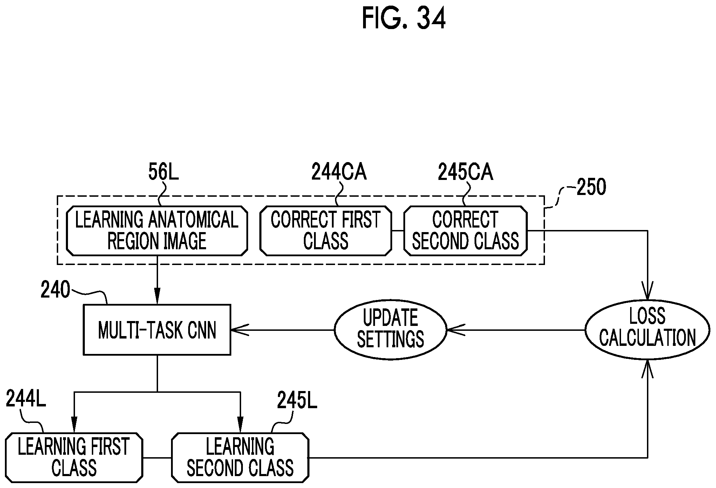

In a fourth embodiment illustrated in Fig. 33 and Fig. 34, a compression unit 241 of a multi-task CNN for class discrimination (hereinafter, abbreviated as multi-task CNN) 240 is used as a feature amount derivation model 246.

As illustrated in Fig. 33 as an example, the multi-task CNN 240 includes a compression unit 241 and an output unit 242. The anatomical region image 56 is input to the compression unit 241. The compression unit 241 converts the anatomical region image 56 into the feature amount set 243. The compression unit 241 transmits the feature amount set 243 to the output unit 242. The output unit 242 outputs two classes of a first class 244 and a second class 245 based on the feature amount set 243. In Fig. 33, the output unit 242 outputs, as the first class 244, a determination result indicating whether dementia is developed or not developed. Further, in Fig. 33, the output unit 242 outputs, as the second class 245, the age of the patient P.

As illustrated in Fig. 34 as an example, the multi-task CNN 240 is trained by inputting learning data 250 in a learning phase before the compression unit 241 is adapted as the feature amount derivation model 246. The learning data 250 is a set of the learning anatomical region image 56L and a correct first class 244CA and a correct second class 245CA corresponding to the learning anatomical region image 56L. The correct first class 244CA is a result obtained by actually determining, by the doctor, whether or not dementia is developed on the learning anatomical region image 56L. In addition, the correct second class 245CA is the actual age of the patient P whose the head MRI image 15 is imaged, the head MRI image 15 being an image from which the learning anatomical region image 56L is obtained.

In the learning phase, the learning anatomical region image 56L is input to the multi-task CNN 240. The multi-task CNN 240 outputs a learning first class 244L and a learning second class 245L in response to the learning anatomical region image 56L. The loss calculation of the multi-task CNN 240 is performed based on the learning first class 244L and the learning second class 245L, and the correct first class 244CA and the correct second class 245CA. In addition, update setting of various coefficients of the multi-task CNN 240 is performed according to a result of the loss calculation, and the multi-task CNN 240 is updated according to the update setting.

In the learning phase of the multi-task CNN 240, while exchanging the learning data 250, a series of processing including inputting of the learning anatomical region image 56L to the multi-task CNN 240, outputting of the learning first class 244L and the learning second class 245L from the multi-task CNN 240, the loss calculation, the update setting, and updating of the multi-task CNN 240 is repeatedly performed. The repetition of the series of processing is ended in a case where prediction accuracy of the learning first class 244L and the learning second class 245L with respect to the correct first class 244CA and the correct second class 245CA reaches a predetermined setting level. The compression unit 241 of the multi-task CNN 240 of which the prediction accuracy reaches the setting level is stored in the storage 20, and is used as the feature amount derivation model 246. Similarly to the second embodiment and the third embodiment, even in the present embodiment, the feature amount set 243 which is output from the compression unit 241 is treated as "feature amount data" according to the technique of the present disclosure.

As described above, in the fourth embodiment, the compression unit 241 of the multi-task CNN 240 is used as the feature amount derivation model 246. The multi-task CNN 240 performs more complicated processing of outputting a plurality of classes (the first class 244 and the second class 245) as compared with the AE 80, the AE 200, the single-task CNN 81, or the single-task CNN 220. For this reason, there is a high possibility that the feature amount set 243 output from the compression unit 241 more comprehensively represents a feature of the anatomical region image 56. Therefore, as a result, it is possible to further improve the prediction accuracy of the dementia opinion.

The first class 244 may be, for example, a degree of progression of dementia in five levels. In addition, the second class 245 may be a determination result of the age group of the patient P. The multi-task CNN 240 may output three or more classes.

In the first embodiment, instead of the single-task CNN 81, the multi-task CNN 240 according to the present embodiment may be used.

[Fifth Embodiment]

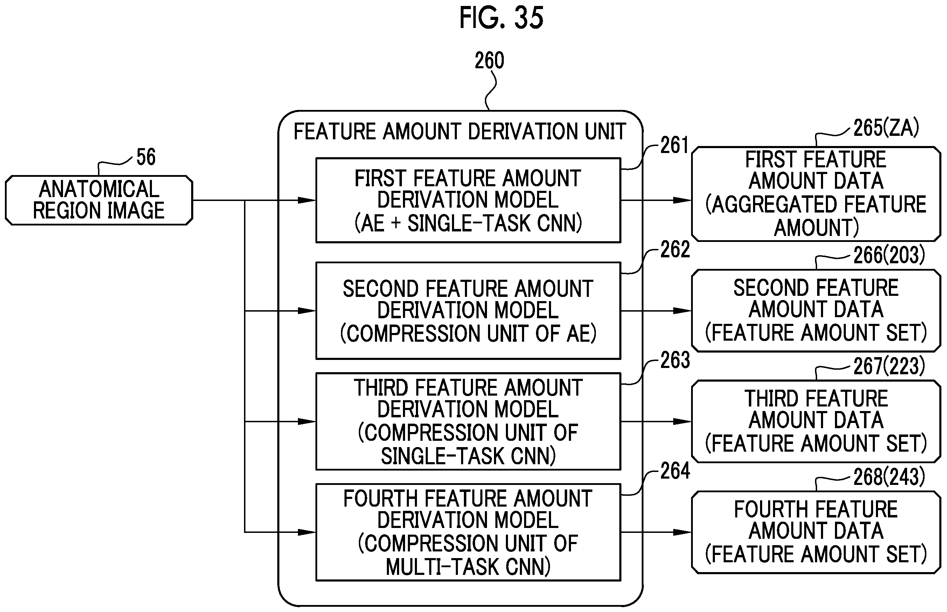

In a fifth embodiment illustrated in Fig. 35, one anatomical region image 56 is input to a plurality of different feature amount derivation models 261 to 264.

As illustrated in Fig. 35 as an example, the feature amount derivation unit 260 according to the present embodiment inputs the one anatomical region image 56 to the first feature amount derivation model 261, inputs the one anatomical region image 56 to the second feature amount derivation model 262, inputs the one anatomical region image 56 to the third feature amount derivation model 263, and inputs the one anatomical region image 56 to the fourth feature amount derivation model 264. Thereby, the feature amount derivation unit 260 outputs first feature amount data 265 from the first feature amount derivation model 261, outputs second feature amount data 266 from the second feature amount derivation model 262, outputs third feature amount data 267 from the third feature amount derivation model 263, and outputs fourth feature amount data 268 from the fourth feature amount derivation model 264.

The first feature amount derivation model 261 is obtained by combining the AE 80 according to the first embodiment and the single-task CNN 81. Therefore, the first feature amount data 265 is the aggregated feature amount ZA. The second feature amount derivation model 262 is obtained by adapting the compression unit 201 of the AE 200 according to the second embodiment. Therefore, the second feature amount data 266 is the feature amount set 203. The third feature amount derivation model 263 is obtained by adapting the compression unit 221 of the single-task CNN 220 according to the third embodiment. Therefore, the third feature amount data 267 is the feature amount set 223. The fourth feature amount derivation model 264 is obtained by adapting the compression unit 241 of the multi-task CNN 240 according to the fourth embodiment. Therefore, the fourth feature amount data 268 is the feature amount set 243.

As described above, in the fifth embodiment, the feature amount derivation unit 260 inputs one anatomical region image 56 to the first feature amount derivation model 261, the second feature amount derivation model 262, the third feature amount derivation model 263, and the fourth feature amount derivation model 264. In addition, the first feature amount data 265, the second feature amount data 266, the third feature amount data 267, and the fourth feature amount data 268 are output from each of the models 261 to 264. Therefore, as compared with a case where one type of feature amount derivation model 37 is used, a wide variety of feature amount data can be obtained. As a result, it is possible to further improve the prediction accuracy of the dementia opinion.

The plurality of different feature amount derivation models may be, for example, a combination of the second feature amount derivation model 262 obtained by adapting the compression unit 201 of the AE 200 and the third feature amount derivation model 263 obtained by adapting the compression unit 221 of the single-task CNN 220. Alternatively, a combination of the third feature amount derivation model 263 obtained by adapting the compression unit 221 of the single-task CNN 220 and the fourth feature amount derivation model 264 obtained by adapting the compression unit 241 of the multi-task CNN 240 may be used. Further, a combination of the third feature amount derivation model 263, which outputs whether or not dementia is developed as the class 224 and is obtained by adapting the compression unit 221 of the single-task CNN 220, and the third feature amount derivation model 263, which outputs the age group of the patient P as the class 224 and is obtained by adapting the compression unit 221 of the single-task CNN 220, may be used.

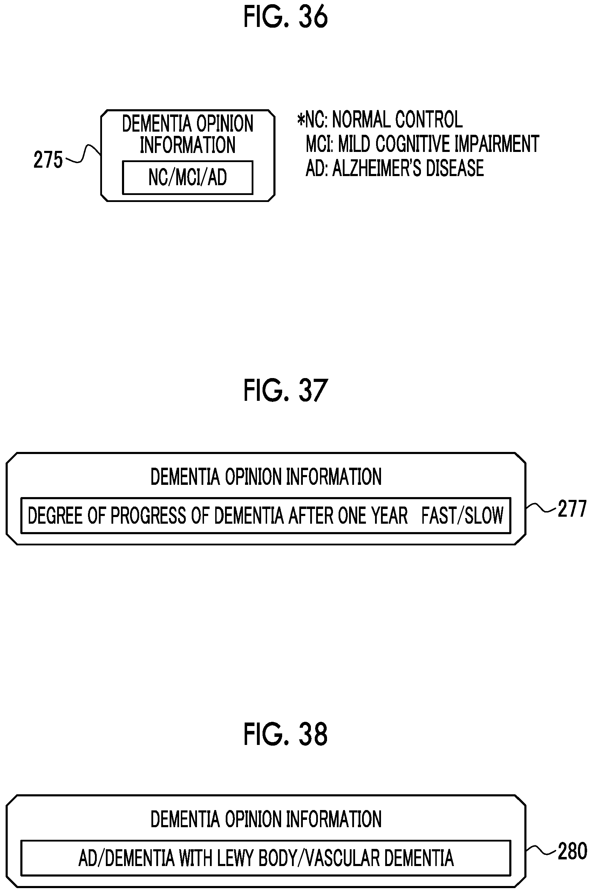

The dementia opinion information is not limited to the contents illustrated in Fig. 8 and the like. For example, as illustrated in the dementia opinion information 275 illustrated in Fig. 36, the dementia opinion information may be any one of normal control (NC), mild cognitive impairment (MCI), and Alzheimer's disease (AD). In addition, for example, as in the dementia opinion information 277 illustrated in Fig. 37, the dementia opinion information may indicate whether a degree of progression of dementia of the patient P one year later is fast or slow. Alternatively, as in the dementia opinion information 280 illustrated in Fig. 38, the dementia opinion information may be a type of dementia, such as Alzheimer's disease, dementia with Lewy body, or vascular dementia.

The learning of the AE 80 and the single-task CNN 81 illustrated in Fig. 14, the learning of the dementia opinion derivation model 39 illustrated in Fig. 16, the learning of the AE 200 illustrated in Fig. 29, the learning of the single-task CNN 220 illustrated in Fig. 32, the learning of the multi-task CNN 240 illustrated in Fig. 34, and the like may be performed by the diagnosis support device 13 or by a device other than the diagnosis support device 13. In addition, the learning may be continuously performed after storing each model in the storage 20 of the diagnosis support device 13.

The PACS server 11 may function as the diagnosis support device 13.

The medical image is not limited to the head MRI image 15 in the example. The medical image may be a positron emission tomography (PET) image, a single photon emission computed tomography (SPECT) image, a computed tomography (CT) image, an endoscopic image, an ultrasound image, or the like.

The organ is not limited to the illustrated brain, and may be a heart, a lung, a liver, or the like. In a case of a lung, right lungs S1 and S2 and left lungs S1 and S2 are extracted as the anatomical regions. In a case of a liver, a right lobe, a left lobe, a gall bladder, and the like are extracted as the anatomical regions. In addition, the disease is not limited to the exemplified dementia, and may be a heart disease, a diffuse lung disease such as interstitial pneumonia, or a dyshepatia such as hepatocirrhosis.

The image is not limited to a medical image. In addition, the target region is not limited to an anatomical region of an organ. Further, the machine learning model is not limited to a model of outputting an opinion of a disease such as dementia. In short, the technique of the present disclosure can be widely applied to multimodal learning in which a plurality of types of data are input as input data of a machine learning model.

In each of the embodiments, for example, as a hardware structure of the processing unit that executes various processing, such as the RW control unit 45, the normalization unit 46, the extraction unit 47, the feature amount derivation units 48 and 260, the dementia opinion derivation units 49 and 210, and the display control unit 50, the following various processors may be used. The various processors include, as described above, the CPU 22 which is a general-purpose processor that functions as various processing units by executing software (an operation program 30), a programmable logic device (PLD) such as a field programmable gate array (FPGA) which is a processor capable of changing a circuit configuration after manufacture, a dedicated electric circuit such as an application specific integrated circuit (ASIC) which is a processor having a circuit configuration specifically designed to execute specific processing, and the like.

One processing unit may be configured by one of these various processors, or may be configured by a combination of two or more processors having the same type or different types (for example, a combination of a plurality of FPGAs and/or a combination of a CPU and an FPGA). Further, the plurality of processing units may be configured by one processor.

As an example in which the plurality of processing units are configured by one processor, firstly, as represented by a computer such as a client and a server, a form in which one processor is configured by a combination of one or more CPUs and software and the processor functions as the plurality of processing units may be adopted. Secondly, as represented by system on chip (SoC), there is a form in which a processor that realizes the functions of the entire system including a plurality of processing units with one integrated circuit (IC) chip is used. As described above, the various processing units are configured by using one or more various processors as a hardware structure.

Further, as the hardware structure of the various processors, more specifically, an electric circuit (circuitry) in which circuit elements such as semiconductor elements are combined may be used.

The technique of the present disclosure can also appropriately combine the various embodiments and/or the various modification examples. In addition, the technique of the present disclosure is not limited to each embodiment, and various configurations may be adopted without departing from the scope of the present disclosure. Further, the technique of the present disclosure extends to a program and a storage medium for non-temporarily storing the program.

The described contents and the illustrated contents are detailed explanations of a part according to the technique of the present disclosure, and are merely examples of the technique of the present disclosure. For example, the descriptions related to the configuration, the function, the operation, and the effect are descriptions related to examples of a configuration, a function, an operation, and an effect of a part according to the technique of the present disclosure. Therefore, it goes without saying that, in the described contents and illustrated contents, unnecessary parts may be deleted, new components may be added, or replacements may be made without departing from the spirit of the technique of the present disclosure. Further, in order to avoid complications and facilitate understanding of the part according to the technique of the present disclosure, in the described contents and illustrated contents, descriptions of technical knowledge and the like that do not require particular explanations to enable implementation of the technique of the present disclosure are omitted.

In this specification, "A and/or B" is synonymous with "at least one of A or B". That is, "A and/or B" means that only A may be included, that only B may be included, or that a combination of A and B may be included. Further, in this specification, even in a case where three or more matters are expressed by being connected using "and/or", the same concept as "A and/or B" is applied.

All documents, patent applications, and technical standards mentioned in this specification are incorporated herein by reference to the same extent as in a case where each document, each patent application, and each technical standard are specifically and individually described by being incorporated by reference.

**Claims**

1. An information processing apparatus comprising:

   a processor; and
   a memory connected to or built in the processor,
   wherein the processor is configured to:

   generate a scatter diagram for a machine learning model that receives a plurality of types of input data and outputs output data according to the input data, by plotting, in a two-dimensional space in which two parameters which are set based on the plurality of types of input data are set as a horizontal axis and a vertical axis, marks representing a plurality of samples obtained by inputting the input data to the machine learning model; and
   display the scatter diagram, the input data, and a type of the output data on a display.

2. The information processing apparatus according to claim 1,
   wherein the processor is configured to:

   display the scatter diagram in a form in which the marks are allowed to be selected; and
   display, in a case where the mark is selected, at least the input data of the sample corresponding to the selected mark.

3. The information processing apparatus according to claim 1 or 2,
   wherein the processor is configured to:
   display pieces of the input data and types of pieces of the output data of at least two samples in a comparable manner.

4. The information processing apparatus according to any one of claims 1 to 3,
   wherein the mark represents the type of the output data.

5. The information processing apparatus according to any one of claims 1 to 4,
   wherein the mark represents matching/mismatching between the output data and an actual result.

**6.** The information processing apparatus according to any one of claims 1 to 5,
wherein the processor is configured to:
set, as the horizontal axis and the vertical axis, the parameters related to two pieces of the input data which are preset among the plurality of types of input data.

**7.** The information processing apparatus according to claim 6,

wherein the machine learning model is constructed by a method of deriving a contribution of each of the plurality of types of input data to the output data, and
the processor is configured to:
set, as the horizontal axis and the vertical axis, the parameters related to pieces of the input data which have a first contribution and a second contribution among the plurality of types of input data.

**8.** The information processing apparatus according to claim 7,
wherein the machine learning model is constructed by a method according to any one of linear discriminant analysis or boosting.

**9.** The information processing apparatus according to any one of claims 1 to 8,
wherein the processor is configured to:
set, as the horizontal axis and the vertical axis, the parameters related to two pieces of the input data which are designated by a user among the plurality of types of input data.

**10.** The information processing apparatus according to any one of claims 1 to 9,
wherein the processor is configured to:
generate the scatter diagram using a t-distributed stochastic neighbor embedding method.

**11.** The information processing apparatus according to any one of claims 1 to 10,
wherein the plurality of types of input data include feature amount data obtained by inputting target region images of a plurality of target regions extracted from an image to feature amount derivation models prepared corresponding to the plurality of target regions, respectively.

**12.** The information processing apparatus according to claim 11,
wherein the feature amount derivation model includes at least one of an auto-encoder, a single-task convolutional neural network for class discrimination, or a multi-task convolutional neural network for class discrimination.

**13.** The information processing apparatus according to claim 11 or 12,

wherein the image is a medical image,
the target regions are anatomical regions of an organ, and
the machine learning model outputs, as the output data, an opinion of a disease.

**14.** The information processing apparatus according to claim 13,
wherein the plurality of types of input data include disease-related information related to the disease.

**15.** The information processing apparatus according to claim 13 or 14,

wherein the organ is a brain, and
the disease is dementia.

**16.** The information processing apparatus according to claim 15,
wherein the anatomical regions include at least one of a hippocampus or a frontotemporal lobe.

**17.** An operation method of an information processing apparatus, the method comprising:

generating a scatter diagram for a machine learning model that receives a plurality of types of input data and outputs output data according to the input data, by plotting, in a two-dimensional space in which two parameters which are set based on the plurality of types of input data are set as a horizontal axis and a vertical axis, marks representing a plurality of samples obtained by inputting the input data to the machine learning model; and

displaying the scatter diagram, the input data, and a type of the output data on a display.

**18.** An operation program of an information processing apparatus, the program causing a computer to execute a process comprising:

generating a scatter diagram for a machine learning model that receives a plurality of types of input data and outputs output data according to the input data, by plotting, in a two-dimensional space in which two parameters which are set based on the plurality of types of input data are set as a horizontal axis and a vertical axis, marks representing a plurality of samples obtained by inputting the input data to the machine learning model; and displaying the scatter diagram, the input data, and a type of the output data on a display.

# FIG. 1

EP 4 270 307 A1

DEMENTIA-RELATED INFORMATION

## FIG. 2

DEMENTIA-RELATED INFORMATION ⌐16

SCORE OF MINI-MENTAL STATE EXAMINATION (MMSE): 26
FAQ: 11
CLINICAL DEMENTIA RATING (CDR): 4
ALZHEIMER'S DISEASE ASSESSMENT SCALE-COGNITIVE
SUBSCALE (ADAS-Cog): 25
AGE: 83
GENOTYPE OF ApoE GENE: ε3/ε3

## FIG. 3

⌐24

| DISPLAY ~17 |
| INPUT DEVICE ~18 |

⟵13

CPU ~22

MEMORY ~21

STORAGE ~20

COMMUNICATION UNIT ~23

# FIG. 4

EP 4 270 307 A1

# FIG. 5

HEAD MRI IMAGE ⌐∼15

46

NORMALIZATION
UNIT

65 ⌐∼ SHAPE NORMALIZATION
PROCESSING

35

66 ⌐∼ SHADE NORMALIZATION
PROCESSING

REFERENCE HEAD
MRI IMAGE

NORMALIZED HEAD
MRI IMAGE ⌐∼55

# FIG. 6

NORMALIZED HEAD MRI IMAGE — 55

EXTRACTION UNIT — 47
SEGMENTATION MODEL — 36

ANATOMICAL REGION IMAGE GROUP — 57

ANATOMICAL REGION IMAGE (LEFT HIPPOCAMPUS) — 56_1

ANATOMICAL REGION IMAGE (RIGHT HIPPOCAMPUS) — 56_2

ANATOMICAL REGION IMAGE (LEFT FRONTOTEMPORAL LOBE) — 56_3

ANATOMICAL REGION IMAGE (RIGHT FRONTOTEMPORAL LOBE) — 56_4

EP 4 270 307 A1

## FIG. 7

## FIG. 8

DEMENTIA OPINION DERIVATION UNIT 49

16 — DEMENTIA-RELATED INFORMATION

ZAG — AGGREGATED FEATURE AMOUNT GROUP

ZA_1 — AGGREGATED FEATURE AMOUNT (LEFT HIPPOCAMPUS)

ZA_2 — AGGREGATED FEATURE AMOUNT (RIGHT HIPPOCAMPUS)

ZA_3 — AGGREGATED FEATURE AMOUNT (LEFT FRONTOTEMPORAL LOBE)

ZA_4 — AGGREGATED FEATURE AMOUNT (RIGHT FRONTOTEMPORAL LOBE)

DEMENTIA OPINION DERIVATION MODEL

QUANTILE NORMALIZATION UNIT 70

LINEAR DISCRIMINANT ANALYSIS UNIT 71

39

58

DEMENTIA OPINION INFORMATION

REMAIN MCI AFTER TWO YEARS (sMCI)/ PROGRESS FROM MCI TO AD AFTER TWO YEARS (cMCI)

*MCI: MILD COGNITIVE IMPAIRMENT
AD: ALZHEIMER'S DISEASE

EP 4 270 307 A1

# FIG. 9

*Z: FEATURE AMOUNT
N: NUMBER OF FEATURE AMOUNTS

56 ANATOMICAL REGION IMAGE

80

82 COMPRESSION UNIT

84 AE FEATURE AMOUNT SET
Z1,Z2,···ZN

83 RESTORATION UNIT

85 RESTORATION IMAGE

81

87 CLASS
sMCI/cMCI

86 OUTPUT UNIT

SINGLE-TASK CNN

37 FEATURE AMOUNT DERIVATION MODEL

ZA AGGREGATED FEATURE AMOUNT → TO DEMENTIA OPINION DERIVATION MODEL

EP 4 270 307 A1

# FIG. 10

$$k = az + by + cx + dw + ev + fu + gt + hs + ir$$

conv

## FIG. 11

## FIG. 12

# FIG. 13

FEATURE AMOUNT SET — 84

86

OUTPUT UNIT

SA MECHANISM LAYER — 110

GAP LAYER — 111

FC LAYER — 112

SMF LAYER — 113

PCA LAYER — 114

CLASS — 87

AGGREGATED FEATURE AMOUNT — ZA

EP 4 270 307 A1

# FIG. 14

# FIG. 15

FIG. 16

# FIG. 17

SAMPLE INFORMATION — 40

57L — LEARNING ANATOMICAL REGION IMAGE GROUP

16L — LEARNING DEMENTIA-RELATED INFORMATION

ZAGL — LEARNING AGGREGATED FEATURE AMOUNT GROUP

58L — LEARNING DEMENTIA OPINION INFORMATION

MATCHING/MISMATCHING INFORMATION — 130

## FIG. 18

DEMENTIA OPINION DERIVATION MODEL —— 39

↓

135

CONTRIBUTION INFORMATION

| ITEM | LEFT HIPPOCAMPUS | RIGHT HIPPOCAMPUS | LEFT FRONTOTEMPORAL LOBE | RIGHT FRONTOTEMPORAL LOBE | MMSE | FAQ | CDR | ADAS-Cog | AGE | GENOTYPE OF ApoE GENE |
|---|---|---|---|---|---|---|---|---|---|---|
| CONTRIBUTION | 0.02 | 0.38 | 0.01 | 0.04 | 0.06 | 0.18 | 0.21 | 0.01 | 0.02 | 0.07 |

↓

42 ——

AXIS SETTING INFORMATION

HORIZONTAL AXIS: AGGREGATED FEATURE AMOUNT (RIGHT HIPPOCAMPUS)
VERTICAL AXIS: CDR

EP 4 270 307 A1

# FIG. 19

SAMPLE INFORMATION — 40

⋮

LEARNING DEMENTIA-RELATED INFORMATION — 16L

⋮

CDR: 4

⋮

LEARNING AGGREGATED FEATURE AMOUNT GROUP

⋮

AGGREGATED FEATURE AMOUNT (RIGHT HIPPOCAMPUS): 100

⋮

ZAGL

LEARNING DEMENTIA OPINION INFORMATION

cMCI

58L

MATCHING/MISMATCHING INFORMATION

MATCHING

130

⋮

AXIS SETTING INFORMATION

HORIZONTAL AXIS: AGGREGATED FEATURE AMOUNT (RIGHT HIPPOCAMPUS)
VERTICAL AXIS: CDR

42

140

Y(CDR)

141D   141B   141C   141A

X (AGGREGATED FEATURE AMOUNT (RIGHT HIPPOCAMPUS))

■ ⋯ sMCI      □ ⋯ cMCI

●○MARK ⋯ MATCHING ✖✖ MARK ⋯ MISMATCHING

142

SAMPLE INFORMATION — 40

⋮

LEARNING DEMENTIA-RELATED INFORMATION — 16L

⋮

CDR: 0.5

⋮

LEARNING AGGREGATED FEATURE AMOUNT GROUP

⋮

AGGREGATED FEATURE AMOUNT (RIGHT HIPPOCAMPUS): 1000

⋮

ZAGL

LEARNING DEMENTIA OPINION INFORMATION

sMCI

58L

MATCHING/MISMATCHING INFORMATION

MISMATCHING

130

⋮

EP 4 270 307 A1

# FIG. 20

150

DIAGNOSIS SUPPORT APPLICATION

PATIENT: TARO FUJI          IMAGING DATE: 08.24.2020  9:55

15S          15A          15C

151          151  ANALYSIS  152          151

# FIG. 21

## FIG. 22

**DIAGNOSIS SUPPORT APPLICATION ~VERIFICATION SCREEN~**

| ITEM | LEFT HIPPOCAMPUS | RIGHT HIPPOCAMPUS | LEFT FRONTOTEMPORAL LOBE | RIGHT FRONTOTEMPORAL LOBE | MMSE | FAQ | CDR | ADAS-Cog | AGE | GENOTYPE OF ApoE GENE |
|---|---|---|---|---|---|---|---|---|---|---|
| CONTRIBUTION | 0.02 | 0.38 | 0.01 | 0.04 | 0.06 | 0.18 | 0.21 | 0.01 | 0.02 | 0.07 |

**TARGET SAMPLE**

LEFT HIPPOCAMPUS

RIGHT HIPPOCAMPUS

LEFT FRONTOTEMPORAL LOBE

RIGHT FRONTOTEMPORAL LOBE

DEMENTIA TEST
MMSE: 26
FAQ: 11
CDR: 4.5
ADAS-Cog: 25
AGE: 83
ApoE GENE TEST: ε3/ε4

OPINION: PROGRESS TO AD AFTER TWO YEARS

Y(CDR)

X (RIGHT HIPPOCAMPUS)

■ ··· REMAIN MCI AFTER TWO YEARS   □ ··· PROGRESS TO AD AFTER TWO YEARS

●○ MARK ··· CORRECT PREDICTION   ✕✕ MARK ··· INCORRECT PREDICTION

*SELECT MARK OF SAMPLE YOU WANT TO COMPARE WITH TARGET SAMPLE.

CLOSE

# FIG. 23

DIAGNOSIS SUPPORT APPLICATION ~VERIFICATION SCREEN~

| ITEM | LEFT HIPPOCAMPUS | RIGHT HIPPOCAMPUS | LEFT FRONTOTEMPORAL LOBE | RIGHT FRONTOTEMPORAL LOBE | MMSE | FAQ | CDR | ADAS-Cog | AGE | GENOTYPE OF ApoE GENE |
|---|---|---|---|---|---|---|---|---|---|---|
| CONTRIBUTION | 0.02 | 0.38 | 0.01 | 0.04 | 0.06 | 0.18 | 0.21 | 0.01 | 0.02 | 0.07 |

## TARGET SAMPLE ~166 (left)

LEFT HIPPOCAMPUS — RIGHT HIPPOCAMPUS

LEFT FRONTOTEMPORAL LOBE — RIGHT FRONTOTEMPORAL LOBE

DEMENTIA TEST
MMSE: 26
FAQ: 11
CDR: 4.5 ~166
ADAS-Cog: 25
AGE: 83
ApoE GENE TEST: ε 3/ ε 4

OPINION: PROGRESS TO AD AFTER TWO YEARS

## 140

Y(CDR)

56_1L

161

168

X (RIGHT HIPPOCAMPUS)

■ ··· REMAIN MCI AFTER TWO YEARS   □ ··· PROGRESS TO AD AFTER TWO YEARS

●○ MARK ··· CORRECT PREDICTION   ✕✕ MARK ··· INCORRECT PREDICTION

142

## TARGET SAMPLE ~166 (right)

LEFT HIPPOCAMPUS — RIGHT HIPPOCAMPUS

LEFT FRONTOTEMPORAL LOBE — RIGHT FRONTOTEMPORAL LOBE

DEMENTIA TEST
MMSE: 28
FAQ: 13
CDR: 4.5 ~166
ADAS-Cog: 22
AGE: 78
ApoE GENE TEST: ε 2/ ε 4

OPINION: PROGRESS TO AD AFTER TWO YEARS

PREDICTION: CORRECT

CLOSE ~167

EP 4 270 307 A1

# FIG. 24

START

ST100
READ HEAD MRI IMAGE AND DEMENTIA-RELATED INFORMATION

ST110
PERFORM NORMALIZATION PROCESSING OF MATCHING HEAD MRI IMAGE WITH REFERENCE HEAD MRI IMAGE

ST120
EXTRACT PLURALITY OF ANATOMICAL REGION IMAGES OF BRAIN FROM HEAD MRI IMAGE

ST130
INPUT ANATOMICAL REGION IMAGES TO FEATURE AMOUNT DERIVATION MODELS AND OUTPUT AGGREGATED FEATURE AMOUNTS FROM FEATURE AMOUNT DERIVATION MODELS

ST140
INPUT AGGREGATED FEATURE AMOUNT GROUP TO DEMENTIA OPINION DERIVATION MODEL AND OUTPUT DEMENTIA OPINION INFORMATION FROM DEMENTIA OPINION DERIVATION MODEL

ST150
DISPLAY DEMENTIA OPINION INFORMATION

ST160
IS INSTRUCTION FOR VERIFICATION OF DEMENTIA OPINION INFORMATION RECEIVED? — NO

YES

ST170
GENERATE VERIFICATION SCREEN INCLUDING SCATTER DIAGRAM

ST180
DISPLAY VERIFICATION SCREEN

END

# FIG. 25

BOOSTING

180

DEMENTIA OPINION
DERIVATION MODEL

CONTRIBUTION
INFORMATION

181

## FIG. 26

185

### DIAGNOSIS SUPPORT APPLICATION ~AXIS SETTING SCREEN~

#### SELECT HORIZONTAL AXIS OF SCATTER DIAGRAM.

188 — 186

- ○ LEFT HIPPOCAMPUS
- ○ LEFT FRONTOTEMPORAL LOBE
- ○ MMSE
- ○ CDR
- ○ AGE

- ○ RIGHT HIPPOCAMPUS
- ◉ RIGHT FRONTOTEMPORAL LOBE
- ○ FAQ
- ○ ADAS-Cog
- ○ GENOTYPE OF ApoE GENE

#### SELECT VERTICAL AXIS OF SCATTER DIAGRAM.

189 — 187

- ○ LEFT HIPPOCAMPUS
- ○ LEFT FRONTOTEMPORAL LOBE
- ○ MMSE
- ○ CDR
- ◉ AGE

- ○ RIGHT HIPPOCAMPUS
- ○ RIGHT FRONTOTEMPORAL LOBE
- ○ FAQ
- ○ ADAS-Cog
- ○ GENOTYPE OF ApoE GENE

190 — [ OK ]     [ CANCEL ] — 191

140

Y (AGE)

X (AGGREGATED FEATURE AMOUNT
(RIGHT FRONTOTEMPORAL LOBE))

FIG. 27

| MMSE OF ALL SAMPLES | FAQ OF ALL SAMPLES | CDR OF ALL SAMPLES | ADAS-Cog OF ALL SAMPLES | AGE OF ALL SAMPLES | GENOTYPE OF ApoE GENE OF ALL SAMPLES |
|---|---|---|---|---|---|
| AGGREGATED FEATURE AMOUNT (LEFT HIPPOCAMPUS) OF ALL SAMPLES | AGGREGATED FEATURE AMOUNT (RIGHT HIPPOCAMPUS) OF ALL SAMPLES | | AGGREGATED FEATURE AMOUNT (RIGHT FRONTOTEMPORAL LOBE) OF ALL SAMPLES | | AGGREGATED FEATURE AMOUNT (LEFT FRONTOTEMPORAL LOBE) OF ALL SAMPLES |

⇓

t-DISTRIBUTED STOCHASTIC NEIGHBOR EMBEDDING METHOD

⇓

140

Y (t-SNE2)

X (t-SNE1)

# FIG. 28

56 — ANATOMICAL REGION IMAGE

200

AE

201 — COMPRESSION UNIT

FEATURE AMOUNT DERIVATION MODEL — 205

FEATURE AMOUNT SET — 203

202 — RESTORATION UNIT

RESTORATION IMAGE — 204

# FIG. 29

LEARNING ANATOMICAL REGION IMAGE — 56L

200 — AE

LEARNING RESTORATION IMAGE — 204L

UPDATE SETTINGS

LOSS CALCULATION

FIG. 30

# FIG. 31

56 ANATOMICAL REGION IMAGE

220

SINGLE-TASK CNN

221 COMPRESSION UNIT

225

FEATURE AMOUNT DERIVATION MODEL

FEATURE AMOUNT SET 223

222 OUTPUT UNIT

CLASS

DEMENTIA IS DEVELOPED/DEMENTIA IS NOT DEVELOPED 224

# FIG. 32

56L         224CA

230

┌─────────────────────────────────────────────┐
│ LEARNING ANATOMICAL    CORRECT │
│ REGION IMAGE          CLASS │
└─────────────────────────────────────────────┘

220

SINGLE-TASK CNN ← UPDATE SETTINGS ← LOSS CALCULATION

LEARNING CLASS

224L

# FIG. 33

56 — ANATOMICAL REGION IMAGE

240

MULTI-TASK CNN

241 — COMPRESSION UNIT ----→ FEATURE AMOUNT DERIVATION MODEL   246

FEATURE AMOUNT SET — 243

242 — OUTPUT UNIT

FIRST CLASS

DEMENTIA IS DEVELOPED/DEMENTIA IS NOT DEVELOPED

244

SECOND CLASS

AGE OF PATIENT

245

FIG. 34

EP 4 270 307 A1

FIG. 35

FEATURE AMOUNT DERIVATION UNIT ~260

ANATOMICAL REGION IMAGE ~56

FIRST FEATURE AMOUNT DERIVATION MODEL (AE + SINGLE-TASK CNN) ~261

SECOND FEATURE AMOUNT DERIVATION MODEL (COMPRESSION UNIT OF AE) ~262

THIRD FEATURE AMOUNT DERIVATION MODEL (COMPRESSION UNIT OF SINGLE-TASK CNN) ~263

FOURTH FEATURE AMOUNT DERIVATION MODEL (COMPRESSION UNIT OF MULTI-TASK CNN) ~264

FIRST FEATURE AMOUNT DATA (AGGREGATED FEATURE AMOUNT) ~265(ZA)

SECOND FEATURE AMOUNT DATA (FEATURE AMOUNT SET) ~266(203)

THIRD FEATURE AMOUNT DATA (FEATURE AMOUNT SET) ~267(223)

FOURTH FEATURE AMOUNT DATA (FEATURE AMOUNT SET) ~268(243)

# FIG. 36

275 ~

DEMENTIA OPINION
INFORMATION

NC/MCI/AD

*NC: NORMAL CONTROL
 MCI: MILD COGNITIVE IMPAIRMENT
 AD: ALZHEIMER'S DISEASE

# FIG. 37

DEMENTIA OPINION INFORMATION

DEGREE OF PROGRESS OF DEMENTIA AFTER ONE YEAR   FAST/SLOW

~277

# FIG. 38

DEMENTIA OPINION INFORMATION

AD/DEMENTIA WITH LEWY BODY/VASCULAR DEMENTIA

~280

## INTERNATIONAL SEARCH REPORT

International application No.

**PCT/JP2021/048387**

### A. CLASSIFICATION OF SUBJECT MATTER

*G06T 7/00*(2017.01)i; *G16H 30/00*(2018.01)i; *G16H 50/20*(2018.01)i; *G06N 20/00*(2019.01)i; *A61B 5/00*(2006.01)i
FI:    A61B5/00 D; G16H30/00; G16H50/20; G06N20/00; G06T7/00 350B; G06T7/00 612

According to International Patent Classification (IPC) or to both national classification and IPC

### B. FIELDS SEARCHED

Minimum documentation searched (classification system followed by classification symbols)

A61B5/00-5/01, A61B5/055, G06N20/20, G06T7/00-7/90, G16H30/00-30/40, G16H50/00-50/80

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2004-188026 A (OLYMPUS CORP) 08 July 2004 (2004-07-08) paragraphs [0026],[0032], [0034], [0046], [0049], [0114], [0117]-[0119], [0226]-[0227], fig. 1, 8, 22, 25-26, 40-41 | 1, 3-6, 9, 11, 13-14, 17-18 |
| Y | | 2, 10, 12, 15-16 |
| A | | 7-8 |
| Y | JP 2014-140521 A (PANASONIC HEALTHCARE CO LTD) 07 August 2014 (2014-08-07) paragraphs [0011]-[0012], [0026], [0030], [0059]-[0060], fig. 1, 5, 9 | 2 |
| Y | US 2019/0347567 A1 (GENETIC INTELLIGENCE, INC.) 14 November 2019 (2019-11-14) paragraph [0023] | 10 |
| Y | US 2016/0148376 A1 (SAMSUNG ELECTRONICS CO., LTD.) 26 May 2016 (2016-05-26) paragraphs [0042], [0046]-[0048], fig. 1 | 12 |
| Y | JP 2020-192068 A (HITACHI LTD) 03 December 2020 (2020-12-03) paragraph [0008] | 15-16 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **09 March 2022** | **22 March 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2021/048387**

| Patent document cited in search report | | | Publication date (day/month/year) | Patent family member(s) | | | Publication date (day/month/year) |
|---|---|---|---|---|---|---|---|
| JP | 2004-188026 | A | 08 July 2004 | US | 2005/0207645 | A1 | |
| | | | | paragraphs [0085], [0086], [0092], [0094], [0106], [0109], [0180], [0183]-[0186], [0305]-[0306], fig. 1, 8, 22, 25-26, 40-41 | | | |
| | | | | WO | 2004/052188 | A1 | |
| | | | | EP | 1570777 | A1 | |
| | | | | CN | 1725975 | A | |
| | | | | AU | 2003289292 | A1 | |
| JP | 2014-140521 | A | 07 August 2014 | (Family: none) | | | |
| US | 2019/0347567 | A1 | 14 November 2019 | WO | 2019/178291 | A1 | |
| US | 2016/0148376 | A1 | 26 May 2016 | KR | 10-2016-0063128 | A | |
| JP | 2020-192068 | A | 03 December 2020 | US | 2020/0380676 | A1 | |
| | | | | paragraph [0008] | | | |

Form PCT/ISA/210 (patent family annex) (January 2015)

**REFERENCES CITED IN THE DESCRIPTION**

**Patent documents cited in the description**

- JP 2019530116 A

**Non-patent literature cited in the description**

- **LAURENS VAN DER MAATEN.** Journal of Machine Learning Research. *Visualizing data using t-SNE,* November 2008